# EUROPEAN PATENT APPLICATION

(11) **EP 4 368 610 A1**
(43) Date of publication of application: **15.05.2024**
(21) Application number: 22852155.5
(22) Date of filing: 02.08.2022
(51) Int. Cl.: C07D 209/02, C07D 213/16, C07D 217/02, A61K 31/403, A61K 31/44, A61K 31/472, A61P 31/12, A61P 31/14

(54) **3CL PROTEASE SMALL-MOLECULE INHIBITOR FOR TREATING OR PREVENTING CORONAVIRUS INFECTION, AND USE THEREOF**

(30) Priority: 02.08.2021 CN 202110882817; 02.11.2021 WO PCT/CN2021/128089
(71) Applicant: The Global Health Drug Discovery Institute, Beijing 100192 (CN)
(72) Inventor: LIU, Renhe, Beijing 100192 (CN); XU, Younong, Seattle, Washington 98125 (US); HUA, Lan, Beijing 100192 (CN); ZHOU, Jingjing, Beijing 100192 (CN); DENG, Hongjing, Beijing 100192 (CN); CHU, Xinjie, Needham, Massachusetts 02494 (US); DING, Sheng, Orinda, California 94563 (US)
(74) Representative: Manitz Finsterwald Patent- und Rechtsanwaltspartnerschaft mbB
(86) International application number: PCT/CN2022/109599
(87) International publication number: WO 2023/011443

(57) **Abstract**

The present invention relates to a 3CL protease small-molecule inhibitor for treating or preventing a coronavirus infection, and the use thereof. Specifically, the present invention relates to a compound of formula (I) or an isotopically labeled compound thereof, or an optical isomer, a geometric isomer, a tautomer, or an isomer mixture thereof, or a pharmaceutically acceptable salt thereof, or a prodrug thereof, or a metabolite thereof, and the use thereof in the preparation of a drug for treating or preventing a coronavirus infection or a disease or symptom caused by a coronavirus.

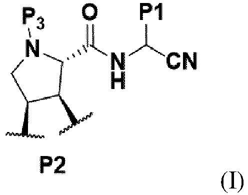

## Description

### CROSS-REFERENCE TO RELATED APPLICATIONS

The present disclosure claims the priorities to Chinese Patent Application No. CN202110882817.6, filed on August 02, 2021, and PCT Patent Application No. PCT/CN2021/128089, filed on November 02, 2021, which are incorporated herein by reference in their entirety.

### TECHNICAL FIELD

The present disclosure relates to the field of biomedicine, and specifically relates to a 3CL protease small-molecule inhibitor for treating or preventing a coronavirus infection and use thereof.

### BACKGROUND

Severe acute respiratory syndrome coronavirus 2 (SARS-CoV-2) is a positive-strand RNA virus that has the largest genome among the currently known RNA viruses. As revealed in the studies, SARS-CoV-2 mainly infects cells via the respiratory mucosal system of humans. After SARS-CoV-2 enters the cells, its viral gene is cleaved by proteases to start translation and replication of required proteins. 3CL proteases identify specific enzyme cleavage sites and cleave a polyprotein precursor into multiple non-structural proteins, and thus are vital to life cycles of viruses and are excellent targets for antiviral drugs. Many marketed antiviral drugs target, for example, 3CL proteases of HIV and HCV. Herein, lopinavir and ritonavir, which are the inhibitors of HIV 3CL protease, interact with a 3CL protease of SARS-CoV-2 in vitro, but they have been proved to be lack of positive effects on patients with SARS-CoV-2 infections in clinical tests. Therefore, it is crucial to develop a strong inhibitor specific for the 3CL protease of SARS-CoV-2.

### SUMMARY

In the first aspect, the present disclosure provides a compound of formula (I) or an isotope-labeled compound thereof, or an optical isomer, a geometric isomer, a tautomer, or an isomer mixture thereof, or a pharmaceutically acceptable salt thereof, or a prodrug thereof, or a metabolite thereof; 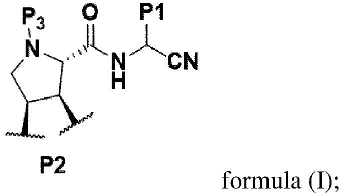 where:
P1 is a five- to ten-membered heteroaryl group that is unsubstituted or optionally substituted by a group R1, where the heteroaryl group contains at least one heteroatom selected from N, O, or S;
P2 together with two adjacent carbon atoms jointly form a three-membered, four-membered, five-membered, or six-membered cycloalkyl group or heterocycloalkyl group that is unsubstituted or optionally substituted by a C₁-C₃ alkyl group or halogen, where the heterocycloalkyl group contains at least one heteroatom selected from N, O, or S;
P3 is selected from 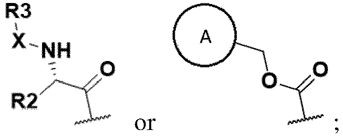
further, where:
R1 is selected from hydrogen, deuterium, halogen, a cyano group, an oxo group, a nitro group, a C₁-C₅ alkyl group, a C₁-C₃ haloalkyl group, a C₁-C₄ alkoxy group, a C₅-C₁₀ aryl group, or a five- to ten-membered heteroaryl group;
R2 is selected from a C₁-C₅ alkyl group, a C₁-C₃ haloalkyl group, a C₃-C₆ cycloalkyl group, a C₁-C₄ alkoxy group, or a C₅-C₁₀ aryl group;
R3 is selected from a C₁-C₅ alkyl group, a C₁-C₃ haloalkyl group, a C₃-C₆ cycloalkyl group, a C₁-C₄ alkoxy group, a C₅-C₁₀ aryl group, or a five- to ten-membered heteroaryl group;
ring A is selected from a C₅-C₁₀ aryl group or a five- to ten-membered heteroaryl group that is unsubstituted or optionally substituted by halogen, where the heteroaryl group contains at least one heteroatom selected from N, O, or S; and
X is selected from

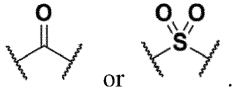

In an embodiment of the present disclosure, the compound of formula (I) may specifically have the following formula (II):

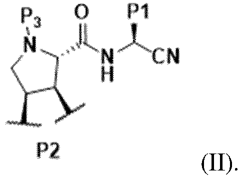

In an embodiment of the present disclosure, the compound of formula (I) may specifically have the following formula (III):

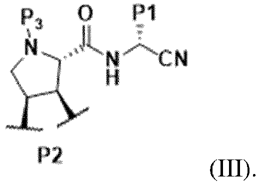

In another embodiment of the present disclosure,
P1 may be selected from a five- to six-membered monocyclic or eight- to ten-membered bicyclic heteroaryl group that is unsubstituted or optionally substituted by a group R1, where the heteroaryl group contains one to three heteroatoms selected from N, O, or S;
P2 together with two adjacent carbon atoms can jointly form a three-membered, four-membered, five-membered, or six-membered cycloalkyl group or heterocycloalkyl group that is unsubstituted or optionally substituted by a methyl group, an ethyl group, fluorine, or chlorine, where the heterocycloalkyl group contains one or two heteroatoms selected from N, O, or S; and
P3 may be selected from 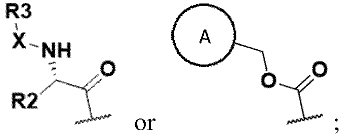
further, where:
R1 may be selected from hydrogen, deuterium, halogen, a cyano group, an oxo group, a nitro group, a C₁-C₅ alkyl group, a C₁-C₃ haloalkyl group, a C₁-C₄ alkoxy group, a C₅-C₁₀ aryl group, or a five- to ten-membered heteroaryl group;
R2 may be selected from a C₁-C₅ alkyl group, a C₁-C₃ haloalkyl group, a C₃-C₆ cycloalkyl group, a C₁-C₄ alkoxy group, or a C₅-C₁₀ aryl group;
R3 may be selected from a C₁-C₅ alkyl group, a C₁-C₃ haloalkyl group, a C₃-C₆ cycloalkyl group, a C₁-C₄ alkoxy group, a C₅-C₁₀ aryl group, or a five- to ten-membered heteroaryl group;
ring A may be selected from a phenyl group, a pyridyl group, a pyrimidinyl group, a pyrrolyl group, a furyl group, or a thienyl group that is unsubstituted or optionally substituted by halogen; and
X may be selected from

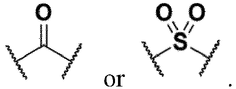

In another embodiment of the present disclosure,
P1 may be selected from 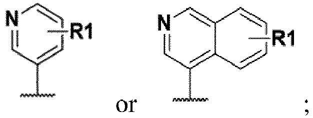
P2 may be selected from 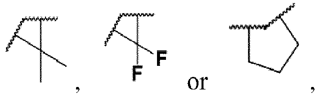 and P2 together with two adjacent carbon atoms jointly form a cyclopropyl group substituted by two methyl groups, a cyclopropyl group substituted by two fluorine atoms, or a cyclopentyl group;
P3 may be selected from 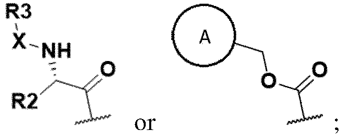
further, where:
R1 may be selected from hydrogen, deuterium, halogen, a cyano group, a nitro group, a C₁-C₅ alkyl group, a C₁-C₃ haloalkyl group, a C₁-C₄ alkoxy group, a C₅-C₁₀ aryl group, or a five- to ten-membered heteroaryl group;
R2 may be selected from a C₁-C₅ alkyl group, a C₁-C₃ haloalkyl group, a C₃-C₆ cycloalkyl group, a C₁-C₄ alkoxy group, or a C₅-C₁₀ aryl group;
R3 may be selected from a C₁-C₅ alkyl group, a C₁-C₃ haloalkyl group, a C₃-C₆ cycloalkyl group, a C₁-C₄ alkoxy group, a C₅-C₁₀ aryl group, or a five- to ten-membered heteroaryl group;
ring A may be selected from a phenyl group that is unsubstituted or optionally substituted by fluorine, chlorine, or bromine; and
X may be selected from

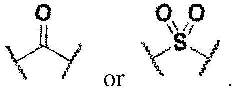

In a preferable embodiment of the present disclosure,
R1 may be selected from hydrogen, deuterium, halogen, a cyano group, a nitro group, a methyl group, an ethyl group, a propyl group, an isopropyl group, a halomethyl group, a haloethyl group, a halopropyl group, a methoxy group, an ethoxy group, a propoxy group, a tert-butoxy group, a phenyl group, a halophenyl group, or a pyridyl group;
R2 may be selected from a methyl group, an ethyl group, a propyl group, an isopropyl group, a butyl group, a tert-butyl group, a halomethyl group, a haloethyl group, a halopropyl group, a cyclopropyl group, a cyclobutyl group, a cyclopentyl group, a cyclohexyl group, a methoxy group, an ethoxy group, a propoxy group, a tert-butoxy group, a phenyl group, or a halophenyl group; and
R3 may be selected from a methyl group, an ethyl group, a propyl group, an isopropyl group, a butyl group, a tert-butyl group, a halomethyl group, a haloethyl group, a halopropyl group, a cyclopropyl group, a cyclobutyl group, a cyclopentyl group, a cyclohexyl group, a methoxy group, an ethoxy group, a propoxy group, a tert-butoxy group, a phenyl group, a halophenyl group, or a pyridyl group.

In a preferable embodiment of the present disclosure,
R1 may be selected from hydrogen, bromine, a cyano group, a methyl group, a phenyl group, a benzyl group, a pyridyl group, or a pyrazolyl group;
R2 may be selected from an isopropyl group, a tert-butyl group, a phenyl group, or a chlorophenyl group; and
R3 may be selected from a methyl group, a trifluoromethyl group, a cyclopropyl group, a tert-butoxy group, a tolyl group, a chlorophenyl group, an aminophenyl group, or an amidophenyl group.

In a preferable embodiment of the present disclosure, the compound of formula (I) may specifically have the following formula: 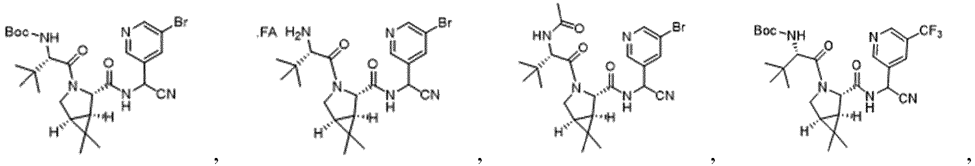 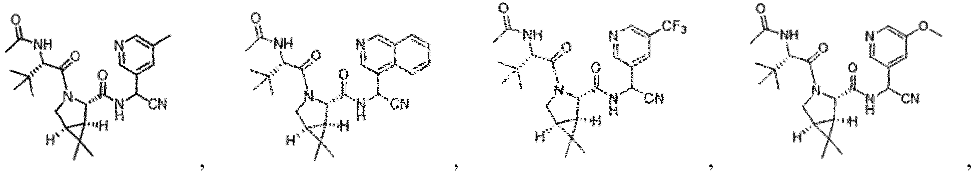 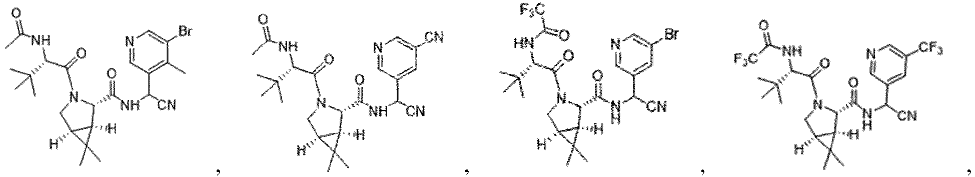 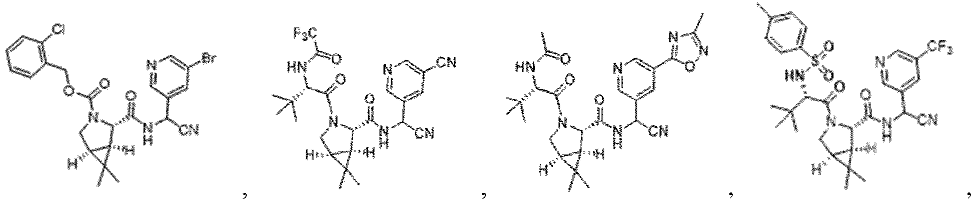 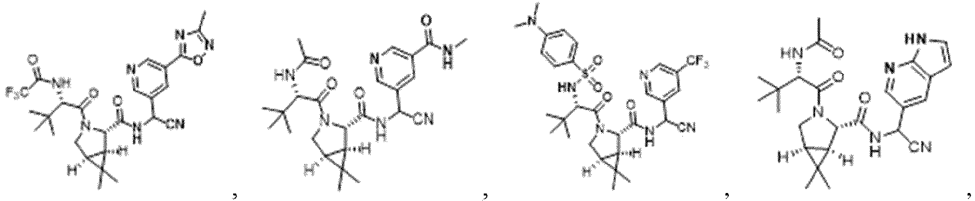 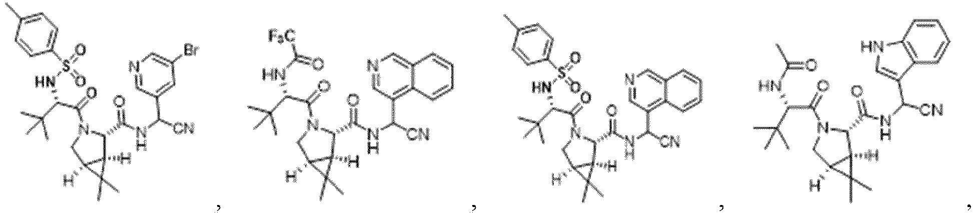 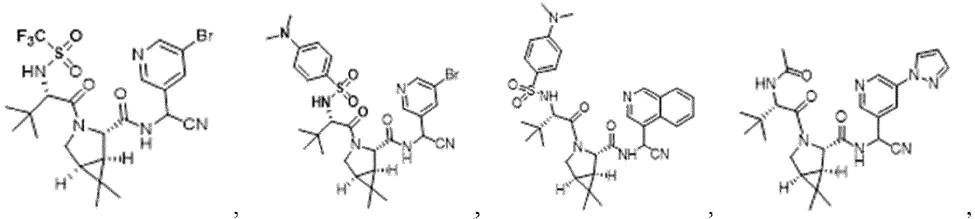 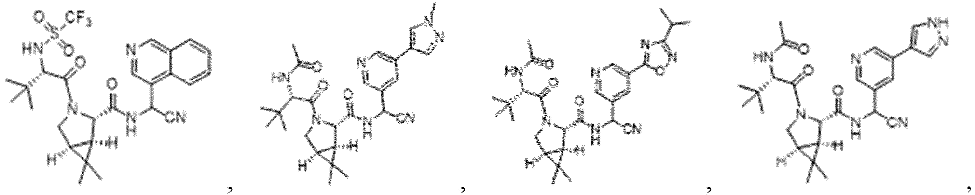 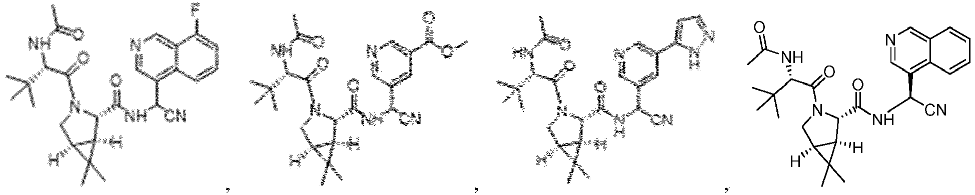 or

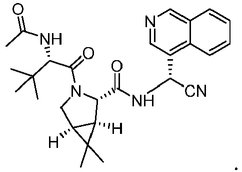

For brevity, the "compound of formula (I)" or the "compound in the present disclosure" described below can also cover any isotope-labeled compound, optical isomer, geometric isomer, tautomer, isomer mixture, pharmaceutically acceptable salt, prodrug or metabolite of the compound of formula (I).

The term "optical isomer" means that when a compound has one or more chiral centers, each chiral center can have an R configuration or an S configuration, and various isomers formed by them are optical isomers. The optical isomers include all diastereoisomers, enantiomers, mesoforms, racemates, or mixtures thereof. For example, the optical isomer can be separated via a chiral chromatographic column or chiral synthesis.

The term "geometric isomer" means that when there is a double bond in the compound, the compound may have a cis isomer, a trans isomer, an E isomer and a Z isomer. The geometric isomer includes a cis isomer, a trans isomer, an E isomer, a Z isomer, or a mixture thereof.

The term "tautomer" refers to an isomer generated due to rapid movement of a specific atom in a molecule between two positions. Those skilled in the art can understand that tautomers can transform into each other, and may coexist when an equilibrium state is reached in a specific state.

Unless otherwise clarified, the "compound of formula (I)" or the "compound in the present disclosure" mentioned herein also covers an isotope-labeled compound obtained by substituting any atom in the compound with its isotopic atom. The present disclosure includes all pharmaceutically acceptable isotope-labeled compounds of the compound of formula (I), where one or more atoms are substituted by an atom having the same atomic number as an atom usually found in nature but a different atomic mass or mass quantity.

Examples of isotopes suitable for inclusion in the compound in the present disclosure include isotopes of hydrogen, such as ²H(D) and ³H(T); isotopes of carbon, such as ¹¹C, ¹³C, and ¹⁴C; isotopes of chlorine, such as ³⁶C1; isotopes of fluorine, such as ¹⁸F; isotopes of iodine such as ¹²³I and ¹²⁵I; isotopes of nitrogen such as ¹³N and ¹⁵N; isotopes of oxygen, such as ¹⁵O, ¹⁷O, and ¹⁸O; and isotopes of sulfur such as ³⁵S.

The isotope-labeled compound of formula (I) can generally be prepared by conventional techniques known to those skilled in the art or by using a suitable isotope-labeled reagent in place of a previously used non-labeled reagent in a method analogous to that described in examples and preparations appended herein.

The compound of formula (I) may exist in a form of pharmaceutically acceptable salt such as acid addition salt and/or base addition salt of the compound of formula (I). Unless otherwise clarified, the "pharmaceutically acceptable salt" used herein includes acid addition salt or base addition salt that may exist in the compound of formula (I).

The pharmaceutically acceptable salt of the compound of formula (I) includes the acid addition salt and base addition salt of the compound of formula (I). A suitable acid addition salt is formed by an acid that forms a non-toxic salt. Examples of the acid addition salt include, but are not limited to: acetate, adipate, aspartate, benzoate, benzenesulfonate, bicarbonate/carbonate, bisulfate/sulfate, borate, camphorsulfonate, citrate, cyclamate, edisylate, formate, fumarate, glucoheptonate, gluconate, glucuronic acid salt, hexafluorophosphate, 2-(4-hydroxybenzyl)benzoate, hydrochloride/chloride, hydrobromide/bromide, hydroiodide/iodide, 2-isethionate, lactate, malate, maleate, malonate, methanesulfonate, methylsulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate, and trifluoroacetate. A suitable base addition salt is formed by a base that forms a non-toxic salt. Examples of the base addition salt include, but are not limited to, aluminum, arginine, calcium, choline, diethylamine, diethanolamine, glycine, lysine, magnesium, meglumine, ethanolamine, potassium, sodium, tromethamine, and zinc salt. Half-salts of acids and bases such as hemisulfate and hemicalcium salts may also be formed. For a review of suitable salt, refer to "Handbook of Pharmaceutical Salts: Properties, Selection and Use (Stahl and Wermuth, Wiley-VCH, 2002). A method for preparing the pharmaceutically acceptable salt of the compound described herein is known to those skilled in the art.

Some compounds in the present disclosure can exist in an unsolvated form and a solvated form (including a hydrated form). In general, the compounds of formula (I) fall within the scope of the present disclosure regardless of whether the compounds exist in the solvated or unsolvated form.

Some compounds in the present disclosure may exist in different crystalline or amorphous forms, and the compounds of formula (I) fall within the scope of the present disclosure regardless of which form the compounds are in.

To avoid ambiguity, definitions of terms used herein are provided below. Unless otherwise clarified, the terms used herein have the same meanings as those described blow.

The term "pharmaceutically acceptable" means that a corresponding compound, carrier or molecule is suitable for administration to humans. Preferably, the term modifies a product approved by any national regulatory agency such as CFDA (PRC), EMEA (Europe) or FDA (USA) for administration to mammals, preferably, humans.

The "prodrug" refers to a derivative that is converted into the compound in the present disclosure via an enzyme-catalyzed reaction such as oxidation, reduction or hydrolysis with an enzyme, gastric acid, or the like under a physiological condition in vivo.

The "metabolite" refers to all molecules originated from any compound in the present disclosure in a cell or an organism, preferably, humans.

The term "cyano group" refers to -CN.

A term "substituted" used herein means that one or more (preferably, 1 to 5, or more preferably, 1 to 3) hydrogen atoms in a group are independently replaced by a corresponding number of substituents.

A term "independently" used herein means that when the number of substituents is greater than one, these substituents may be the same or different.

A term "optional" or "optionally" used herein means that an event modified by the term may or may not occur. For example, a group "optionally substituted" means that the group may be unsubstituted or substituted.

A term "heteroatom" used herein represents oxygen (O), nitrogen (N), or S(O)ₘ (m can be 0, 1, or 2, namely, a sulfur atom S, a sulfoxide group SO, or a sulfonyl group S(O)₂).

A term "alkyl group" used herein refers to saturated aliphatic hydrocarbon, including straight and branched chains. In some embodiments, an alkyl group has 1-8, 1-6 or 1-3 carbon atoms. For example, the term "Ci-Cs alkyl group" refers to a straight or branched chain radical having 1 to 8 carbon atoms. A definition of the term "Ci-Cs alkyl group" includes terms "C₁-C₆ alkyl group", "C₁-C₃ alkyl group" and "C₁-C₄ alkyl group". Examples of the alkyl group include, but are not limited to, a methyl group, an ethyl group, a propyl group, an isopropyl group, a butyl group, an isobutyl group, a sec-butyl group, a tert-butyl group, a pentyl group, a 2-pentyl group, a 3-pentyl group, an isopentyl group, a neopentyl group, an (R)-2-methylbutyl group, an (S)-2-methylbutyl group, a 3-methylbutyl group, a 2,3-dimethylpropyl group, a 2,3-dimethylbutyl group, and a hexyl group. The alkyl group is optionally substituted with one or more (for example, 1 to 5) suitable substituents.

A term "n-membered heterocycloalkyl group" used herein refers to a cycloalkyl group having m ring-forming carbon atoms and (n-m) ring-forming heteroatoms, where the heteroatom is selected from O, S and N. For example, three- to seven-membered heterocycloalkyl groups include, but are not limited to, oxetane, thietane, azetidine, tetrahydrofuran, tetrahydrothiophene, pyrrolidine, tetrahydropyran, tetrahydrothiopyran, piperidine, morpholine, piperazine, oxepane, thiepane and azepane. The heterocycloalkyl group is optionally substituted with one or more suitable substituents.

A term "C₅-C₇ aryl group" used herein refers to an aryl group having an aromatic ring including 5-7 carbon atoms, or preferably, a phenyl group.

A term "n-membered heteroaryl group" used herein refers to a heteroaryl group having m carbon atoms forming an aromatic ring And (n-m) heteroatoms forming an aromatic ring, where the heteroatom is selected from O, S and N. For example, five- to seven-membered heteroaryl groups include, but are not limited to, pyrazine, pyrazole, pyrrole, furan, thiophene, thiazole, and pyridine. The heteroaryl group is optionally substituted with one or more suitable substituents.

The term "C₇-C₁₁ bicyclic aryl group" used herein refers to a bicyclic aryl group having 7-11 carbon atoms, for example, naphthalene or indene. The bicyclic aryl group is optionally substituted with one or more suitable substituents.

A term "n-membered bicyclic heteroaryl group" used herein refers to a bicyclic heteroaryl group having m carbon atoms forming a dual aromatic ring And (n-m) heteroatoms forming a dual aromatic ring, where the heteroatom is selected from O, S and N. For example, seven- to eleven-membered bicyclic heteroaryls include, but are not limited to, quinoline, isoquinoline and benzothiazole. The bicyclic heteroaryl group is optionally substituted with one or more suitable substituents.

A term "haloalkyl group" used herein refers to an alkyl group having one or more halogen substituents (at most a perhaloalkyl group, that is, each hydrogen atom of the alkyl group is substituted by a halogen atom). For example, a term "C₁-C₆ haloalkyl group" refers to a C₁-C₆ alkyl group having one or more halogen substituents (at most a perhaloalkyl group, that is, each hydrogen atom of the alkyl group is substituted by a halogen atom). For another example, the term "C₁-C₄ haloalkyl group" refers to a C₁-C₄ alkyl group (up to a perhaloalkyl group, that is, each hydrogen atom of the alkyl group is substituted with a halogen atom) having one or more halogen substituents; the term "C₁-C₃ haloalkyl group" refers to a C₁-C₃ alkyl group (up to the perhaloalkyl group, that is, each hydrogen atom of the alkyl group is substituted with a halogen atom) having one or more halogen substituents; and the term "C₁-C₂ haloalkyl group" refers to a C₁-C₂ alkyl group (that is, the methyl group or the ethyl group) (up to the perhaloalkyl group, that is, each hydrogen atom of the alkyl group is substituted with a halogen atom) having one or more halogen substituents. For another example, a term "C₁ haloalkyl group" refers to a methyl group having 1, 2, or 3 halogen substituents. Examples of the haloalkyl group include CF₃, C₂F₅, CHF₂, CH₂F, CH₂CF₃, CH₂Cl, and the like.

Herein, number ranges related to the number of substituents, the number of carbon atoms, and the number of ring Atoms represent enumerations of all integers within the range, and the range is only used as a simplified representation. For example, "1-4 substituents" represent 1, 2, 3, or 4 substituents; and "3-8 ring Atoms" represent 3, 4, 5, 6, 7, or 8 ring Atoms. Therefore, the number ranges related to the number of substituents, the number of carbon atoms, and the number of ring Atoms also cover any one of its sub-ranges, and each sub-range is also construed as being disclosed herein.

The compound in the present disclosure can be prepared in various ways known to those skilled in the field of organic synthesis. With reference to a synthesis route of a specific compound in a specific embodiment of the present disclosure, those skilled in the art can make appropriate adjustments to reactant raw materials and reaction conditions to obtain a synthesis method of another compound.

In the second aspect, the present disclosure provides a pharmaceutical composition, containing the compound of formula (I) or the isotope-labeled compound thereof, or the optical isomer, the geometric isomer, the tautomer, or the isomer mixture thereof, or the pharmaceutically acceptable salt thereof, or the prodrug thereof, or the metabolite thereof, and a pharmaceutically acceptable carrier.

The pharmaceutically acceptable carrier can be an organic or inorganic inert carrier material. For example, appropriate carriers include water, gelatin, gum arabic, lactose, starch, magnesium stearate, talc, vegetable oil, polyalkylene glycol, petrolatum, mannitol, cellulose, a cellulose derivative, sodium saccharin, glucose, sucrose, magnesium carbonate, saline, glycerin and ethanol. In addition, the pharmaceutical composition may also contain another drug additive, for example, a flavoring Agent, a preservative, a stabilizer, an emulsifier, a buffering Agent, a diluent, a binder, a wetting agent, a disintegrant, a lubricant or a glidant.

A dosage form of the pharmaceutical composition in the present disclosure may be a liquid dosage form, a solid dosage form, or a semisolid dosage form. The liquid dosage form can be a solution (including a true solution and a colloid solution), an emulsion (including o/w type, w/o type and double emulsions), a suspension, an injection (including a water injection, a powder injection and an infusion), eye drops, nasal drops, a lotion, a liniments, and the like; the solid dosage form can be a tablet (including an ordinary tablet, an enteric-coated tablet, a buccal tablet, a dispersible tablet, a chewable tablet, an effervescent tablet and an orally disintegrating tablet), a capsule (including a hard capsule, a soft capsule and an enteric-coated capsule), granules, powder, pills, a suppository, films, patches, an aerosol, a spray, and the like; and the semisolid dosage form can be an ointment, a gel, a paste, and the like. The pharmaceutical composition in the present disclosure can be prepared into a common formulation or a sustained-release formulation, a controlled-release formulation, a targeted formulation, and various particle delivery systems.

In some embodiments, the dosage form of the pharmaceutical composition includes a tablet, granules, powder, syrup, an inhalation, and an injection.

The solid dosage form administered orally may include a capsule, a tablet, pills, powder and granules. In such solid dosage form, the active compound is mixed with at least one inert excipient (or carrier) (for example, sodium citrate or dicalcium phosphate), which may also include: (a) a filler or a blending agent (for example, starch, lactose, sucrose, glucose, mannitol and silicic acid); (b) a binder (for example, carboxymethylcellulose, alginate, a gel, polyvinylpyrrolidone, sucrose and gum arabic); (c) a humectant (for example, glycerol); (d) a disintegrant (for example, agar-agar, calcium carbonate, potatO, or tapioca starch, alginic acid, some synthetic silicates and sodium carbonate); (e) a solution blocker (for example, paraffin); (f) an absorption enhancer (for example, a quaternary ammonium compound); (g) a wetting agent (for example, cetyl alcohol and glyceryl monostearate); (h) an adsorbent (for example, kaolin and bentonite) and (i) a lubricant (for example, talc, calcium stearate, magnesium stearate, polyethylene glycol solid and sodium lauryl sulfate) or a mixture thereof.

The formulations suitable for parenteral administration such as injections may include aqueous and nonaqueous isotonic sterile solutions suitable for injection, and aqueous and nonaqueous sterile suspensions. The parenterally administered formulations provided herein are optionally accommodated in sealed unit-dose or multi-dose containers, such as ampoules, and may be stored in freeze-dried (lyophilized) conditions that require only addition of a sterile liquid carrier (such as water for injection) immediately before use. Examples of suitable diluents for reconstitution of the pharmaceutical composition (for example, before injection) include bacteriostatic water for injection, 5% dextrose in water, phosphate buffered saline, Ringer's (Ringer's) solution, saline, sterile water, deionized water, and a combination thereof.

The spray can contain excipients such as lactose, talc, silicic acid, aluminum hydroxide, calcium silicate and polyamide powder, or a mixture of these substances. The spray can additionally contain customary propellants, for example, chlorofluorocarbons and volatile unsubstituted hydrocarbons, for example, butane and propane. The inhalant can contain an excipient such as lactose, or an aqueous solution containing, for example, polyethylene oxide-9-lauryl ether, glycocholate and deoxycholate, or an oily solution administered in a form of nasal drops, spray, or gel.

The contents of the compounds in the present disclosure in the pharmaceutical composition thereof can be adjusted based on an actual need (for example, a dosage form, an administration method, and an administered object), for example, 0.1wt%-95wt%, for example, 1wt%-95wt%, 5wt%-90wt%, or 10wt%-80wt%.

Specifically, the pharmaceutical composition in the present disclosure may specifically include 0.01-10 g (for example, 0.05 g, 0.1 g, 0.5 g, 1 g or 5 g) of the compound in the present disclosure.

In the third aspect, the present disclosure provides use of the compound of formula (I) or the isotope-labeled compound thereof, or the optical isomer, the geometric isomer, the tautomer, or the isomer mixture thereof, or the pharmaceutically acceptable salt thereof, or the prodrug thereof, or the metabolite thereof in the preparation of a medicament for treating or preventing a coronavirus infection or a disease or symptom caused by a coronavirus in a subject in need. The compound of formula (I) or the isotope-labeled compound thereof, or the optical isomer, the geometric isomer, the tautomer, or the isomer mixture thereof, or the pharmaceutically acceptable salt thereof, or the prodrug thereof, or the metabolite thereof may be used to treat or prevent a coronavirus infection or a disease or symptom caused by a coronavirus in a subject in need.

The term "subject" used in the present disclosure refers to any human or non-human organism that could potentially benefit from treatment with the compound of formula (I). Exemplarily, a subject includes a human or a mammal at any age. Preferably, the subject is a human.

The term "treatment" used herein includes treatment of a disease or a symptom in the mammal, especially the human, and includes: (a) inhibition of an infection, a disease or a symptom, that is, inhibition or delaying of development of the infection, the disease or the symptom; (b) relief of the infection, the disease or the symptom, that is, improvement of the disease or the symptom; and/or (c) cure of the infection, the disease or the symptom.

The term "prevention" used herein includes a prophylactic therapy in the mammal, especially the human, to reduce occurrence possibility of the infection, the disease or the symptom. A patient can be selected for the prophylactic therapy based on a factor of an increased risk of infection or having a disease or a symptom compared to the general population. The "prevention" can include treatment of a subject who has not yet exhibited an infection or a clinical condition, and prevention of a second occurrence of the same or similar infection or clinical condition.

The inventor of the present disclosure have found that the compound in the present disclosure can inhibit the coronavirus infection. For example, the compound can act as a reversible covalent small-molecule inhibitor against 3CL protease of SARS-CoV-2 (namely, a 3CL protease inhibitor), thereby inhibiting replication of the SARS-CoV-2. Therefore, the compound in the present disclosure may be used to prevent or treat the coronavirus infection or the disease or the symptom caused by the coronavirus.

In some embodiments, the coronavirus is selected from severe acute respiratory syndrome coronavirus (SARS-CoV), severe acute respiratory syndrome coronavirus 2 (SARS-CoV-2), Middle East respiratory syndrome coronavirus (MERS-CoV), human coronavirus OC43 (HCoV-OC43), murine hepatitis coronavirus (MHV), and a coronavirus that shares more than 85% homology with any one of the foregoing coronaviruses and that has viral activity. In some embodiments, the coronavirus is the severe acute respiratory syndrome coronavirus 2 (SARS-CoV-2).

In some embodiments, the disease or symptom caused by a coronavirus includes a respiratory infection, severe acute respiratory syndrome (SARS), pneumonia (including severe pneumonia), gastroenteritis (including acute gastroenteritis), cough, fever, shivering, vomiting, headache, chill, shortness of breath, cytokine storm and the like caused by the virus.

In the fourth aspect, the present disclosure provides a method for treating or preventing a coronavirus infection or a disease or symptom caused by a coronavirus, where the method includes giving a therapeutically effective dose of a compound of formula (I) or an isotope-labeled compound thereof, or an optical isomer, a geometric isomer, a tautomer, or an isomer mixture thereof, or a pharmaceutically acceptable salt thereof, or a prodrug thereof, or a metabolite thereof to a subject in need.

In some embodiments, the compound in the present disclosure can be administered orally, parenterally, intravenously, intramuscularly, subcutaneously, nasally, buccally, or ocularly, or via lung, respiratory tract or vagina, or rectally, intraperitoneally, intralesionally, around the lesion, or via another route.

The "therapeutically effective dose" refers to the dose of the compound in the present disclosure that is effective in treating or preventing the coronavirus infection or the disease or symptom caused by the coronavirus when administered alone or in combination.

A specific dose administered depends on the route of administration, severity of the disease, age and weight of the patient, and another factor usually considered by an attending physician when determining the most suitable individual regimen and dosage level for a particular patient. For example, a daily dose of the compound in the present disclosure may be specifically 0.001-150 mg/kg body weight (for example, 0.1 mg/kg body weight, 1 mg/kg body weight, 10 mg/kg body weight, or 100 mg/kg body weight).

Specific administration frequency can be determined by those skilled in the related art, for example, once a day, once in 2 days, once in 3 days, once in 4 days, once in 5 days, once in 6 days, twice a day, or thrice a day.

In the fifth aspect, the present disclosure provides a method for preparing A compound of formula (I) or an isotope-labeled compound thereof, or an optical isomer, a geometric isomer, a tautomer, or an isomer mixture thereof, or a pharmaceutically acceptable salt thereof, specifically comprising: 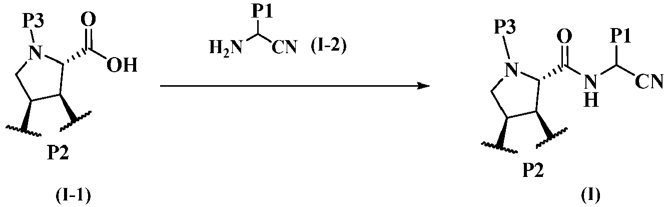 preparing the compound of formula (I) or the isotope-labeled compound thereof, or the optical isomer, the geometric isomer, the tautomer, the isomer mixture thereof, or the pharmaceutically acceptable salt thereof via a condensation reaction between a compound of formula (I-1) or an isotope-labeled compound thereof, or an optical isomer, a geometric isomer, a tautomer, or an isomer mixture thereof, or a pharmaceutically acceptable salt thereof and a compound of formula (I-2), where P1, P2 and P3 have definitions in formula (I).

In a preferable embodiment of the present disclosure, the condensation reaction is carried out in presence of a condensing agent and an alkaline reagent.

In a preferable embodiment of the present disclosure, the condensing agent is selected from one or more of a carbodiimide condensing agent, an onium salt condensing agent and an organophosphorus condensing agent.

In a preferable embodiment of the present disclosure, the condensing agent is selected from one or more of DCC, DIC, EDCI, HOBt, DMAP, HOAt, HATU, HBTU, HCTU, TBTU, BOP, pyBOP, DPP-Cl, DPPA, and BOP-Cl.

In a preferable embodiment of the present disclosure, the alkaline reagent is selected from an organic base or an inorganic base.

In a preferable embodiment of the present disclosure, the alkaline reagent is selected from one or more of DIEA, NMM and triethylamine.

In a preferable embodiment of the present disclosure, the compound of formula (I) or the isotope-labeled compound thereof, or the optical isomer, the geometric isomer, the tautomer, the isomer mixture thereof, or the pharmaceutically acceptable salt thereof is separated to obtain a compound of formula (II) and a compound of formula (III) or an isotope-labeled compound thereof, or an optical isomer, a geometric isomer, a tautomer, or an isomer mixture thereof, or a pharmaceutically acceptable salt thereof:

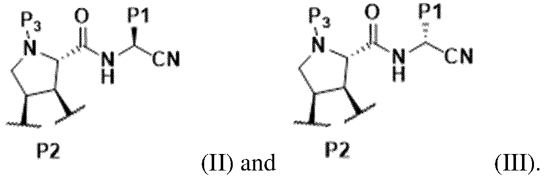

Those skilled in the art can understand that a definition and a preferred item described in one aspect of the present disclosure are also applicable to other aspects. Those skilled in the art can understand that the embodiments of various aspects of the present disclosure can be combined in various manners without departing from the subject and concept of the present disclosure, and these combinations also fall within the scope of the present disclosure.

### BRIEF DESCRIPTION OF DRAWINGS

The accompanying drawings are intended to provide a further understanding of the present disclosure and form a part of the specification, and are used together with the following detailed description to explain the present disclosure, but do not constitute a limitation of the present disclosure. In the accompanying drawings:
FIG. 1 shows a pharmacokinetic (PK) data diagram of compound GDI-001 (15 mg/kg) orally administered to rats;
FIG. 2 shows a pharmacokinetic (PK) data diagram of compound GDI-006 (15 mg/kg) orally administered to rats;
FIG. 3 shows a pharmacokinetic (PK) data diagram of compound GDI-011 (15 mg/kg) orally administered to rats;
FIG. 4 shows a pharmacokinetic (PK) data diagram of compound GDI-012 (15 mg/kg) orally administered to rats; and
FIG. 5 shows a pharmacokinetic (PK) data diagram of compound GDI-014 (15 mg/kg) orally administered to rats.

### DETAILED DESCRIPTION

A compound of formula (I) in the present disclosure can be synthesized in various methods known to those skilled in the field of organic synthesis. The following specific examples provide some exemplary synthetic methods of the compound of formula (I), and these methods are well known in the field of synthetic chemistry. Apparently, with reference to the exemplary solution in this patent, those skilled in the art can easily design another synthetic route for the compound of formula (I) by appropriately adjusting a reactant, a reaction condition and a protecting group.

The present disclosure is further described below with reference to examples, but these examples are not intended to limit the scope of the present disclosure. Unless otherwise stated, all reactants used in the examples are commercially available; and instruments and devices used in synthesis experiments and product analyses and detection are all conventional instruments and devices commonly used in organic syntheses.

### Example 1: Preparation of compound GDI-001 (tert-butyl((2S)-1-((1R,2S,5S)-2-((5-bromopyridin-3-yl)(cyano)methyl)carbamoyl)-6,6-dimethyl-3-azabicyclo[3.1.0]hexan-3-yl)-3,3 -dimethyl-1-oxobutan-2-yl)carbamate)

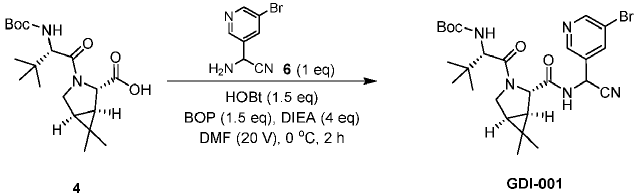

### (1) Synthesis steps of compound GDI-001

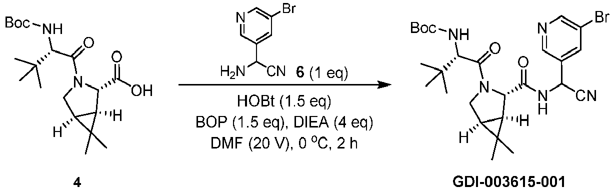

1. DIEA (701 mg, 5.43 mmol, 945 uL, 4eq), HOBt (275 mg, 2.04 mmol, 1.5eq) and BOP (900 mg, 2.04 mmol, 1.5eq) were added to a solution of Cpd.4 (0.50 g, 1.36 mmol, 1eq) and Cpd.6 (288 mg, 1.36 mmol, 1eq) in DMF (2 mL) at 0°C.
2. A resulting mixture was continuously stirred for 2 hours.
3. Appearance of a target product was revealed via LC-MS.
4. A reactant solution was filtered and a filtrate was spin-dried.
5. Prep-HPLC (neutral condition) was used for purification directly.
6. GDI-001 (320 mg, 553 µmol, yield: 40.8%, purity: 97.2%) was obtained as a white solid.

¹H NMR (400 MHz, DMSO-d₆) δ 9.63 - 9.37 (m, 1H), 8.76 (d, *J =* 3.1 Hz, 1H), 8.66 (d, *J* = 7.7 Hz, 1H), 8.09 (s, 1H), 6.68 (t, J = 8.1 Hz, 1H), 6.46 - 6.17 (m, 1H), 4.24 (s, 1H), 4.00 (d, *J* = 9.1 Hz, 1H), 3.94 - 3.76 (m, 2H), 1.60 - 1.49 (m, 1H), 1.35 (s, 10H), 1.31 - 1.22 (m, 1H), 1.06 - 0.98 (m, 3H), 0.90 (s, 11H), 0.91 - 0.83 (m, 10H).

### Example 2: Preparation of compound GDI-002 ((1R,2S,5S)-3-((S)-2-amino-3,3-dimethylbutyryl)-N-((5-bromopyridin-3-yl)(cyano)methyl)-6,6-dimethyl-3-azabicyclo[3.1.0]he xane-2-carboxamide formate)

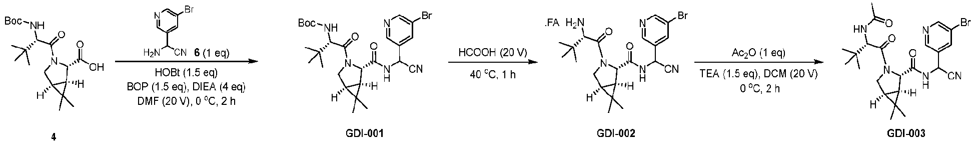

### (1) The operation of step 1 is the same as that in Example 1.

### (2) Synthesis steps of compound GDI-002

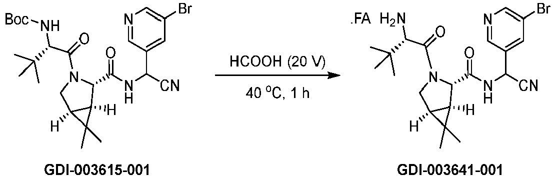

1. GDI-001 (280 mg, 498 µmol, 1eq) was dissolved in HCOOH (5.6 mL) at room temperature.
2. A resulting mixture reacted for 1 hour at 40°C.
3. GDI-001 was completely consumed under monitoring of LC-MS, and a main peak indicated target product MS.
4. The target product was pin-dried directly to obtain a crude solid.
5. The crude solid was diluted with MeCN (10 mL) and H₂O (50 mL) and then lyophilized to remove excess HCOOH to obtain 0.26 g of the crude product of GDI-002.
6. Prep-HPLC (FA condition) was directly used for purification and separation.
7. GDI-002 (0.23 g, 452 µmol, yield: 90.9%, purity: 100%) was obtained as a white solid.

¹H NMR (400 MHz, DMSO-d₆) δ 9.65 - 9.45 (m, 1H), 8.76 (s, 1H), 8.66 (d, *J =* 7.2 Hz, 1H), 8.22 (s, 1H), 8.11 (d, *J* = 10.2 Hz, 1H), 6.49 - 6.22 (m, 1H), 4.24 (s, 1H), 3.88 - 3.75 (m, 2H), 3.70 - 3.62 (m, 1H), 1.63 - 1.50 (m, 1H), 1.41 - 1.24 (m, 1H), 1.10 - 0.99 (m, 4H), 0.95 - 0.89 (m, 5H), 0.88 (s, 9H).

### Example 3: Preparation of compound 3 ((1R,2S,5S)-3-((S)-2-acetamido-3,3-dimethylbutyryl)-N-((5-bromopyridin-3-yl)(cyano)methyl)-6,6-dimethyl-3-azabicyclo[3.1.0]he xane-2-carboxamide)

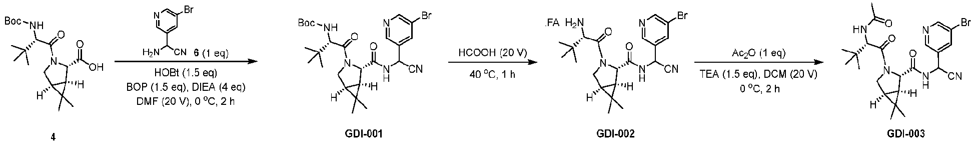

### (1) The operations of step 1 and step 2 are the same as those in Example 2.

### (2) Synthesis steps of compound GDI-003

1. Ac₂O (32.1 mg, 315 µmol, 29.5 uL, 1eq) was added to a solution of GDI-002 (160 mg, 315 µmol, 1eq, FA) and TEA (63.7 mg, 629 µmol, 87.6 uL, 2eq) in DCM (3.2 mL) at 0°C.
2. A resulting mixture was continuously stirred for 2 hours at 0°C.
3. GDI-002 was completely consumed under monitoring of LC-MS, and target product MS appeared.
4. A reactant solution was spin-dried.
5. Prep-HPLC (neutral condition) was directly used for purification.
6. GDI-003 (87 mg, 172 µmol, yield: 54.8%, purity: 100%) was obtained as a pale yellow solid.

¹H NMR (400 MHz, DMSO-d₆) δ 9.64 - 9.42 (m, 1H), 8.76 (d, *J* = 2.1 Hz, 1H), 8.67 (d, *J* = 8.6 Hz, 1H), 8.08 (s, 1H), 7.95 - 7.81 (m, 1H), 6.48 - 6.21 (m, 1H), 4.28 (d, *J* = 8.5 Hz, 1H), 4.21 (d, *J* = 3.1 Hz, 1H), 3.94 - 3.73 (m, 2H), 1.84 (s, 3H), 1.60 - 1.48 (m, 1H), 1.40 - 1.24 (m, 1H), 1.06 - 0.98 (m, 3H), 0.98 - 0.88 (m, 9H), 0.88 - 0.82 (m, 3H).

### Example 4: Preparation of compound GDI-004 (tert-butyl((2S)-1-((1R,2S,5S)-2-((cyano (5-(trifluoromethyl)pyridin-3-yl)methyl)carbamoyl)-6,6-dimethyl-3-azabicyclo[3.1.0]hexan-3-yl)-3,3-dimethyl-1-oxobutan-2-yl)carbamate)

Compound GDI-004 was obtained in a method similar to that in Example 3.

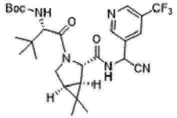

¹H NMR (400 MHz, DMSO-d₆) δ 9.71 - 9.50 (m, 1H), 9.08 - 9.00 (m, 1H), 8.98 (d, *J =* 10.3 Hz, 1H), 8.20 (s, 1H), 6.73 - 6.61 (m, 1H), 6.56 - 6.32 (m, 1H), 4.30 - 4.20 (m, 1H), 4.00 (d, *J* = 9.4 Hz, 1H), 3.94 - 3.79 (m, 2H), 1.60 - 1.51 (m, 1H), 1.35 (s, 9H), 1.32 - 1.21 (m, 1H), 1.06 - 0.99 (m, 3H), 0.97 - 0.82 (m, 12H).

### Example 5: Preparation of compound GDI-005 ((1R,2S,5S)-3-((S)-2-acetamido-3,3-dimethylbutyryl)-N-(cyano(5-methylpyridin-3-yl)methyl)-6,6-dimethyl-3-azabicyclo[3.1.0]hex ane-2-carboxamide)

### (1) Synthesis steps of Cpd.8

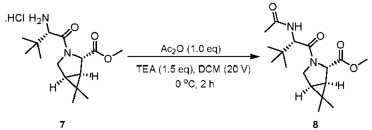

1. (1R,2S,SS)-3-[(2S)-2-amino-3,3-dimethyl-butyryl]-6,6-dimethyl-3-azabicyclo[3.1.0]hexane-2-carboxylate methyl ester (174 mg, 616.20 µmol, 1eq) was dissolved in DCM (3.48 mL) at 0°C.
2. Then TEA (124.70 mg, 1.23 mmol, 171.53 µL, 2eq) was added.
3. Ac₂O (75.49 mg, 739.44 µmol, 69.26 µL, 1.2eq) was added to the foregoing reactant solution in batches.
4. A resulting mixture was continuously stirred for 2 hours at 0°C.
5. A target product was revealed via LC-MS.
6. A saturated NH₄Cl (300 mL) solution was added dropwise.
7. The reactant solution was extracted with EtOAc (300 mL*3).
8. Organic phases were combined and spin-dried.
9. (1R,2S,SS)-3-[(2S)-2-acetylamino-3,3-dimethyl-butyryl]-6,6-dimethyl-3-azabicyclo[3.1.0] hexane-2-carboxylate methyl ester (0.18 g, 537.65 µmol, yield: 87.25%) was obtained as a colorless oily substance.

### (2) Synthesis steps of Cpd.9

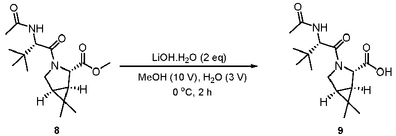

1. Cpd.8 (0.18 g, 554.85 µmol, 1eq) was added to a mixed solution of MeOH (1.8 mL) and H₂O (0.54 mL) at 0°C, followed by LiOH.H₂O (46.57 mg, 1.11 mmol, 2eq).
2. A resulting mixture was continuously stirred for 2 hours at 0°C.
3. Appearance of a target product was revealed via LC-MS.
4. The reaction was quenched with saturated NH₄Cl (100 mL) solution at 0°C.
5. At low temperature, pH was adjusted to 5-6 with 2M citric acid solution.
6. An aqueous phase was extracted with EtOAc (20 mL*3).
7. Organic phases were combined and spin-dried.
8. (1R,2S,SS)-3-[(2S)-2-acetylamino-3,3-dimethyl-butyryl]-6,6-dimethyl-3-azabicyclo[3.1.0] hexane-2-carboxylic acid (130 mg, 418.83 µmol, yield: 75.49%) was obtained a colorless liquid.

### (3) Synthesis steps of compound GDI-005

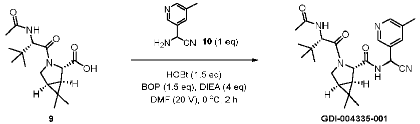

1. DIEA (108.26 mg, 837.66 µmol, 145.91 µL, 4eq), HOBt (42.45 mg, 314.12 µmol, 1.5eq) and BOP (138.93 mg, 314.12 µmol, 1.5eq) were added to a solution of (1R,2S,5S)-3-[(2S)-2-acetylamino-3,3-dimethyl-butyryl]-6,6-dimethyl-3-azabicyclo[3.1.0]hexane-2 -carboxylic acid (65 mg, 209.42 µmol, 1eq) and 2-amino-2-(5-methyl-3-pyridyl)acetonitrile (36.99 mg, 251.30 µmol, 1.2eq) in DMF (1.3 mL) at 0°C.
2. A resulting mixture was continuously stirred for 2 hours.
3. Appearance of a target product was revealed via LC-MS.
4. A reactant solution was filtered and a filtrate was spin-dried.
5. Prep-HPLC (neutral condition) was used for purification directly.
6. GDI-005 (27.1 mg, 61.65 µmol, yield: 29.44%) was obtained as a grayish yellow solid.

¹H NMR (400 MHz, DMSO-d₆) δ 9.57 - 9.33 (m, 1H), 8.55 - 8.37 (m, 2H), 7.87 (t, *J* = 7.6 Hz, 1H), 7.69 (d, *J* = 9.2 Hz, 1H), 6.38 - 6.15 (m, 1H), 4.29 (d, *J* = 8.7 Hz, 1H), 4.23 (s, 1H), 3.93 - 3.73 (m, 2H), 2.33 (s, 3H), 1.84 (s, 3H), 1.64 - 1.46 (m, 1H), 1.40 - 1.19 (m, 1H), 1.05 - 0.97 (m, 3H), 0.93 (s, 9H), 0.89 - 0.82 (m, 3H).

### Example 6: Preparation of compound GDI-006 ((1R,2S,5S)-3-((S)-2-acetamido-3,3-dimethylbutyryl)-N-(cyano(isoquinolin-4-yl)methyl)-6,6-dimethyl-3-azabicyclo[3.1.0]hexane-2 -carboxamide)

Compound GDI-006 was obtained in a method similar to that in Example 5.

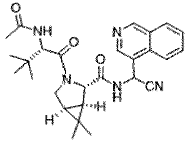

¹H NMR (400 MHz, DMSO-*d*₆) δ 9.59 - 9.44 (m, 1H), 9.42 (s, 1H), 8.72 (d, *J* = 7.0 Hz, 1H), 8.29 - 8.16 (m, 1H), 8.06 - 7.95 (m, 1H), 7.95 - 7.83 (m, 1H), 7.83 - 7.70 (m, 2H), 6.94 - 6.80 (m, 1H), 4.35 - 4.24 (m, 1H), 4.20 (d, *J* = 14.6 Hz, 1H), 3.94 - 3.75 (m, 2H), 1.88 - 1.78 (m, 3H), 1.62 - 1.46 (m, 1H), 1.33 - 1.04 (m, 1H), 1.04 - 0.96 (m, 3H), 0.97 - 0.88 (m, 9H), 0.85 - 0.77 (m, 3H).

### Example 7: Preparation of compound GDI-007 ((1R,2S,5S)-3-((S)-2-acetamido-3,3-dimethylbutanol)-N-(cyano(5-(trifluoromethyl)pyridin-3-yl)methyl)-6,6-dimethyl-3-azabicyclo [3.1.0]hexane-2-carboxamide)

Compound GDI-007 was obtained in a method similar to that in Example 5.

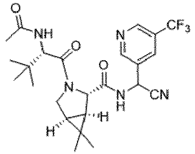

¹H NMR (400 MHz, DMSO-*d*₆) δ 9.36 - 9.17 (m, 1H), 9.07 - 8.93 (m, 1H), 8.20 (s, 1H), 7.97 - 7.74 (m, 1H), 6.61 - 6.31 (m, 1H), 4.31 (dd, *J* = 34.0, 8.7 Hz, 1H), 4.22 (d, *J* = 4.7 Hz, 1H), 3.96 - 3.76 (m, 2H), 1.85 (s, 3H), 1.63 - 1.51 (m, 1H), 1.45 - 1.22 (m, 1H), 1.05 - 1.00 (m, 3H), 0.99 - 0.89 (m, 9H), 0.89 - 0.85 (m, 3H).

### Example 8: Preparation of compound GDI-008 ((1R,2S,5S)-3-((S)-2-acetamido-3,3-dimethylbutyryl)-N-(cyano(5-methoxypyridin-3-yl)methyl)-6,6-dimethyl-3-azabicyclo[3.1.0]he xane-2-carboxamide)

Compound GDI-008 was obtained in a method similar to that in Example 5.

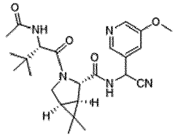

¹H NMR (400 MHz, DMSO-*d*₆) δ 9.56 - 9.38 (m, 1H), 8.39 - 8.21 (m, 2H), 7.95 - 7.79 (m, 1H), 7.43 (s, 1H), 6.42 - 6.18 (m, 1H), 4.29 (d, *J* = 8.6 Hz, 1H), 4.26 - 4.18 (m, 1H), 3.86 (s, 3H), 3.85 - 3.76 (m, 2H), 1.84 (s, 3H), 1.65 - 1.44 (m, 1H), 1.41 - 1.34 (m, 1H), 1.28 - 1.20 (m, 1H), 1.06 - 0.98 (m, 3H), 0.92 (s, 9H), 0.88 - 0.82 (m, 3H).

### Example 9: Preparation of compound GDI-009 ((1R,2S,5S)-3-((S)-2-acetamido-3,3-dimethylbutyryl)-N-((5-bromo-4-methylpyridin-3-yl)(cyano)methyl)-6,6-dimethyl-3-azabicycl o[3.1.0]hexane-2-carboxamide)

Compound GDI-009 was obtained in a method similar to that in Example 5.

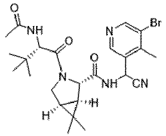

¹H NMR (400 MHz, DMSO-*d*₆) δ 9.44 - 9.15 (m, 1H), 8.83 - 8.72 (m, 1H), 8.63 (s, 1H), 7.92 - 7.78 (m, 1H), 6.50 - 6.35 (m, 1H), 4.26 (t, *J* = 10.1 Hz, 1H), 4.19 - 4.09 (m, 1H), 3.93 - 3.72 (m, 2H), 2.43 - 2.29 (m, 3H), 1.89 - 1.75 (m, 3H), 1.65 - 1.49 (m, 1H), 1.31 - 1.18 (m, 1H), 1.10 - 0.99 (m, 3H), 0.99 - 0.77 (m, 12H).

### Example 10: Preparation of compound GDI-010 ((1R,2S,5S)-3-((S)-2-acetamido-3,3-dimethylbutyryl)-N-(cyano(5-cyanopyridin-3-yl)methyl)-6,6-dimethyl-3-azabicyclo[3.1.0]hexa ne-2-carboxamide)

Compound GDI-010 was obtained in a method similar to that in Example 5.

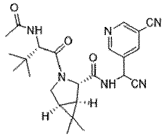

¹H NMR (400 MHz, DMSO-*d*₆) δ 9.68 - 9.48 (m, 1H), 9.07 (d, *J* = 5.0 Hz, 1H), 8.94 (d, *J* = 9.7 Hz, 1H), 8.31 (d, *J* = 7.8 Hz, 1H), 7.88 (d, *J* = 8.4 Hz, 1H), 6.53 - 6.25 (m, 1H), 4.27 (d, *J* = 8.5 Hz, 1H), 4.21 (d, *J* = 4.4 Hz, 1H), 3.95 - 3.75 (m, 2H), 1.85 (s, 3H), 1.65 - 1.48 (m, 1H), 1.43 - 1.30 (m, 1H), 1.07 - 1.00 (m, 3H), 0.91 (s, 9H), 0.88 - 0.83 (m, 3H).

### Example 11: Preparation of compound GDI-011 ((1R,2S,5S)-N-((5-bromopyridin-3-yl) (cyano)methyl)-3-((S)-3,3-dimethyl-2-(2,2,2-trifluoroacetylamino)butyryl)-6,6-dimethyl-3-aza bicyclo[3.1.0]hexane-2-carboxamide)

Compound GDI-011 was obtained in a method similar to that in Example 5.

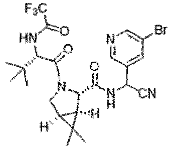

¹H NMR (400 MHz, DMSO-*d*₆) δ 9.66 - 9.46 (m, 1H), 8.76 (s, 1H), 8.67 (d, *J* = 5.3 Hz, 1H), 8.09 (s, 1H), 6.48 - 6.22 (m, 1H), 4.38 (s, 1H), 4.25 (s, 1H), 3.95 - 3.87 (m, 1H), 3.75 - 3.62 (m, 1H), 3.21 - 3.04 (m, 1H), 2.31 - 2.03 (m, 1H), 1.64 - 1.51 (m, 1H), 1.37 - 1.26 (m, 1H), 1.05 - 1.01 (m, 3H), 0.97 (s, 9H), 0.89 - 0.81 (m, 3H).

### Example 12: Preparation of compound GDI-012 ((1R,2S,5S)-N-(cyano(5-(trifluoromethyl) pyridin-3-yl)methyl)-3-((S)-3,3-dimethyl-2-(2,2,2-trifluoroacetylamino)butyryl)-6,6-dimethyl-3 -azabicyclo[3.1.0]hexane-2-carboxamide)

Compound GDI-012 was obtained in a method similar to that in Example 5.

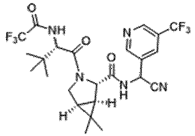

¹H NMR (400 MHz, DMSO-d₆) δ 9.72 - 9.51 (m, 1H), 9.48 - 9.32 (m, 1H), 9.04 (s, 1H), 8.98 (d, *J* = 8.4 Hz, 1H), 8.21 (s, 1H), 6.59 - 6.33 (m, 1H), 4.37 (d, *J* = 7.3 Hz, 1H), 4.25 (s, 1H), 3.99 - 3.80 (m, 1H), 3.68 (t, *J* = 12.0 Hz, 1H), 1.68 - 1.51 (m, 1H), 1.52 - 1.30 (m, 1H), 1.06 - 1.01 (m, 3H), 1.01 - 0.90 (m, 9H), 0.89 - 0.84 (m, 3H).

### Example 13: Preparation of compound GDI-013 (2-chlorobenzyl((1R,2S,5S)-2-((5-bromopyridin-3-yl)(cyano)methyl)carbamoyl)-6,6-dimethyl-3-azabicyclo[3.1.0]hexane-3-carb oxylate)

Compound GDI-013 was obtained in a method similar to that in Example 5.

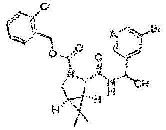

¹H NMR (400 MHz, DMSO-d₆) δ 9.68 - 9.46 (m, 1H), 8.83 - 8.59 (m, 2H), 8.15 - 7.99 (m, 1H), 7.50 - 7.17 (m, 4H), 6.43 - 6.26 (m, 1H), 5.25 - 4.93 (m, 2H), 4.11 (d, *J* = 22.8 Hz, 1H), 3.77 - 3.61 (m, 1H), 3.51 - 3.38 (m, 1H), 1.55 - 1.44 (m, 1H), 1.44 - 1.32 (m, 1H), 1.09 - 0.99 (m, 3H), 0.94 - 0.83 (m, 3H).

### Example 14: Preparation of compound GDI-014 ((1R,2S,5S)-N-(cyano(5-cyanopyridin-3-yl)methyl)-3-((S)-3,3-dimethyl-2-(2,2,2-trifluoroacetylamino)butyryl)-6,6-dimethyl-3-azabicycl o[3.1.0]hexane-2-carboxamide)

Compound GDI-014 was obtained in a method similar to that in Example 5.

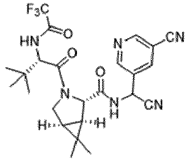

¹H NMR (400 MHz, DMSO-*d*₆) δ 9.74 - 9.52 (m, 1H), 9.50 - 9.32 (m, 1H), 9.11 - 9.03 (m, 1H), 8.94 (d, *J* = 6.0 Hz, 1H), 8.38 - 8.27 (m, 1H), 6.52 - 6.27 (m, 1H), 4.37 (s, 1H), 4.25 (s, 1H), 4.04 - 3.84 (m, 1H), 3.69 (t, *J* = 12.3 Hz, 1H), 1.66 - 1.54 (m, 1H), 1.51 - 1.33 (m, 1H), 1.06 - 1.01 (m, 3H), 1.00 - 0.91 (m, 9H), 0.89 - 0.79 (m, 3H).

### Example 15: Preparation of compound GDI-015 ((1R,2S,5S)-3-((S)-2-acetamido-3,3-dimethylbutyryl)-N-(cyano(5-(3-methyl-1,2,4-oxadiazol-5-yl)pyridin-3-yl)methyl)-6,6-dimethy 1-3-azabicyclo[3.1.0]hexane-2-carboxamide)

Compound GDI-015 was obtained in a method similar to that in Example 5.

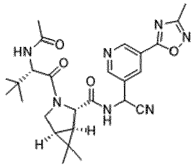

¹H NMR (400 MHz, DMSO-*d*₆) δ 9.72 - 9.51 (m, 1H), 9.32 - 9.24 (m, 1H), 8.93 (d, *J* = 6.8 Hz, 1H), 8.52 (s, 1H), 7.99 - 7.80 (m, 1H), 6.59 - 6.39 (m, 1H), 4.26 (d, *J* = 8.8 Hz, 1H), 4.22 (d, *J* = 6.9 Hz, 1H), 3.96 - 3.73 (m, 2H), 2.47 (s, 3H), 1.84 (s, 3H), 1.59 - 1.48 (m, 1H), 1.41 - 1.25 (m, 1H), 1.05 - 0.99 (m, 3H), 0.97 - 0.82 (m, 12H).

### Example 16: Preparation of compound GDI-016 ((1R,2S,5S)-N-(cyano(5-(trifluoromethyl) pyridin-3-yl)methyl)-3-((S)-3,3-dimethyl-2-((4-methylphenyl)sulfonamido)butanoyl)-6,6-dimet hyl-3-azabicyclo[3.1.0]hexane-2-carboxamide)

Compound GDI-016 was obtained in a method similar to that in Example 5.

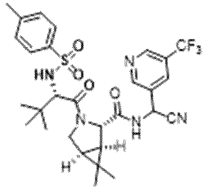

¹H NMR (400 MHz, DMSO-*d*₆) δ 9.68 - 9.55 (m, 1H), 9.02 - 8.91 (m, 2H), 8.18 (d, J = 7.6 Hz, 1H), 7.80 - 7.70 (m, 1H), 7.67 - 7.55 (m, 2H), 7.30 (d, J = 7.9 Hz, 2H), 6.50 - 6.27 (m, 1H), 4.07 (d, J = 5.8 Hz, 1H), 3.77 (td, J = 10.5, 5.6 Hz, 1H), 3.58 (s, 1H), 3.43 (dd, J = 17.4, 10.1 Hz, 1H), 2.32 (s, 3H), 0.95 (d, J = 17.3 Hz, 3H), 0.75 (d, J = 2.8 Hz, 9H), 0.57 (d, J = 22.5 Hz, 3H).

### Example 17: Preparation of compound GDI-017 ((1R,2S,5S)-N-(cyano(5-(3-methyl-1,2,4-oxadiazol-5-yl)pyridin-3-yl)methyl)-3-(S)-3,3-dimethyl-2-(2,2,2-trifluoroacetylamino)butyryl)-6,6-dimethyl-3-azabicyclo[3.1.0]hexane-2-carboxamide)

Compound GDI-017 was obtained in a method similar to that in Example 5.

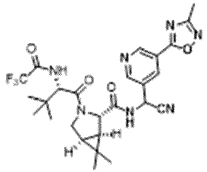

¹H NMR (400 MHz, DMSO-*d*₆) δ 9.67 - 9.62 (m, 1H), 9.40 - 9.32 (m, 1H), 9.23 (dd, J = 3.4, 2.0 Hz, 1H), 8.89 (t, J = 2.6 Hz, 1H), 8.49 (dt, J = 4.8, 2.2 Hz, 1H), 6.53 - 6.38 (m, 1H), 4.33 (t, J = 6.7 Hz, 1H), 4.21 (d, J = 5.1 Hz, 1H), 3.91 - 3.83 (m, 1H), 3.68 - 3.60 (m, 1H), 2.43 (s, 3H), 1.53 (dd, J = 14.9, 6.7 Hz, 1H), 1.39 - 1.27 (m, 1H), 0.99 (d, J = 13.9 Hz, 3H), 0.89 (d, J = 13.8 Hz, 9H), 0.82 (d, J = 5.7 Hz, 3H).

### Example 18: Preparation of compound GDI-018 ((1R,2S,5S)-3-((S)-2-acetamido-3,3-dimethylbutyryl)-N(cyano(5-(methylcarbamoyl)pyridin-3-yl)methyl)-6,6-dimethyl-3-azabicycl o[3.1.0]hexane-2-carboxamide)

Compound GDI-018 was obtained in a method similar to that in Example 5.

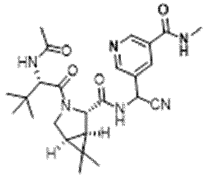

¹H NMR (400 MHz, DMSO-*d*₆) δ 9.56 - 9.45 (m, 1H), 9.00 (t, J = 2.4 Hz, 1H), 8.76 (dt, J = 6.2, 3.2 Hz, 2H), 8.38 - 8.17 (m, 1H), 7.94 - 7.78 (m, 1H), 6.46 - 6.31 (m, 1H), 4.36 - 4.19 (m, 2H), 3.91 - 3.73 (m, 2H), 2.83 (t, J = 4.2 Hz, 3H), 1.85 (s, 3H), 1.59 - 1.49 (m, 1H), 1.38 - 1.23 (m, 1H), 1.02 (d, J = 16.1 Hz, 3H), 0.92 (d, J = 5.8 Hz, 9H), 0.87 (d, J = 12.0 Hz, 3H).

### Example 19: Preparation of compound GDI-019 ((1R,2S,5S)-N-(cyano(5-(trifluoromethyl) pyridin-3-yl)methyl)-3-((S)-2-((4-(dimethylamino)phenyl)sulfonamido)-3,3-dimethylbutanoyl) -6,6-dimethyl-3-azabicyclo[3.1.0]hexane-2-carboxamide)

Compound GDI-019 was obtained in a method similar to that in Example 5.

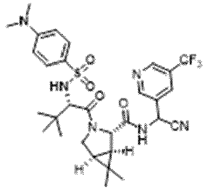

¹H NMR (400 MHz, DMSO-*d*₆) δ 9.70 - 9.54 (m, 1H), 9.00 (d, J = 8 Hz, 1H), 8.93 (d, J = 8 Hz, 1H), 8.21 - 8.15 (m, 1H), 7.51 (d, J = 8.5 Hz, 2H), 7.22 (dd, J = 16.1, 8.8 Hz, 1H), 6.68 (d, J = 8.8 Hz, 2H), 6.51 - 6.27 (m, 1H), 4.10 (d, J = 2.5 Hz, 1H), 3.81 - 3.72 (m, 1H), 3.54 - 3.39 (m, 2H), 2.94 (s, 6H), 1.55 - 1.50 (m, 1H), 1.33 - 1.17 (m, 1H), 0.96 (d, J = 16.4 Hz, 3H), 0.74 (s, 9H), 0.67 (d, J = 17.4 Hz, 3H).

### Example 20: Preparation of compound GDI-020 ((1R,2S,5S)-3-((S)-2-acetamido-3,3-dimethylbutyryl)-N(cyano(1H-pyrrole[2,3-b]pyridin-5-yl)methyl)-6,6-dimethyl-3-azabicyclo[3 .1.0]hexane-2-carboxamide)

Compound GDI-020 was obtained in a method similar to that in Example 5.

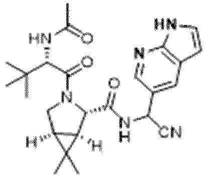

¹H NMR (400 MHz, DMSO-*d*₆) δ 11.83 (s, 1H), 9.38 (t, J = 7.5 Hz, 1H), 8.31 (dd, J = 4.6, 2.2 Hz, 1H), 8.08 (dd, J = 6.8, 2.2 Hz, 1H), 7.88 (t, J = 9.6 Hz, 1H), 7.56 (s, 1H), 6.50 (ddd, J = 8.7, 3.4, 1.8 Hz, 1H), 6.34 - 6.24 (m, 1H), 4.31 (d, J = 8.7 Hz, 1H), 4.25 (s, 1H), 3.91 - 3.74 (m, 2H), 1.84 (d, J = 5.3 Hz, 3H), 1.58 - 1.46 (m, 1H), 1.36 - 1.15 (m, 2H), 1.00 (d, J = 27.2 Hz, 3H), 0.94 (d, J = 2.9 Hz, 9H), 0.85 (d, J = 8.3 Hz, 3H).

### Example 21: Preparation of compound GDI-021 ((1R,2S,5S)-N-((5-bromopyridin-3-yl) (cyano)methyl)-3-((S)-3,3-dimethyl-2-((4-methylphenyl)sulfonamido)butanoyl)-6,6-dimethyl-3 -azabicyclo[3.1.0]hexane-2-carboxamide)

Compound GDI-021 was obtained in a method similar to that in Example 5.

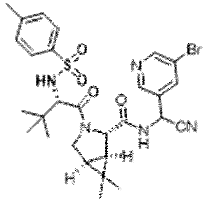

¹H NMR (400 MHz, DMSO-*d*₆) δ 9.66 - 9.50 (m, 1H), 8.80 - 8.62 (m, 2H), 8.09 (dt, J = 4.6, 2.2 Hz, 1H), 7.78 (t, J = 8.0 Hz, 1H), 7.71 - 7.58 (m, 2H), 7.34 (d, J = 8.0 Hz, 2H), 6.45 - 6.21 (m, 1H), 4.11 (d, J = 4.3 Hz, 1H), 3.88 - 3.75 (m, 1H), 3.66 - 3.60 (m, 1H), 3.54 - 3.41 (m, 1H), 2.36 (s, 3H), 1.62 - 1.48 (m, 1H), 1.35 - 1.20 (m, 1H), 0.99 (d, J = 15.4 Hz, 3H), 0.92 - 0.71 (m, 9H), 0.61 (d, J = 17.7 Hz, 3H).

### Example 22: Preparation of compound GDI-022 ((1R,2S,5S)-N-(cyano(isoquinolin-4-yl) methyl)-3-((S)-3,3-dimethyl-2-(2,2,2-trifluoroacetylamino)butyryl)-6,6-dimethyl-3-azabicyclo[ 3.1.0]hexane-2-carboxamide)

Compound GDI-022 was obtained in a method similar to that in Example 5.

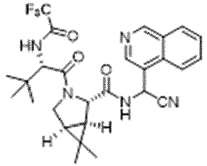

¹H NMR (400 MHz, DMSO-*d*₆) δ 9.62 - 9.46 (m, 1H), 9.44 - 9.30 (m, 2H), 8.73 (d, J = 5.9 Hz, 1H), 8.29 - 8.20 (m, 1H), 8.03 - 7.88 (m, 1H), 7.80 (td, J = 6.7, 3.5 Hz, 2H), 6.92 - 6.83 (m, 1H), 4.40 (t, J = 7.6 Hz, 1H), 4.24 (d, J = 14.3 Hz, 1H), 3.98 - 3.91 (m, 1H), 3.75 - 3.66 (m, 1H), 1.65 - 1.50 (m, 1H), 1.35 (d, J = 7.6 Hz, 1H), 1.08 - 0.90 (m, 12H), 0.81 (d, J = 7.0 Hz, 3H).

### Example 23: Preparation of compound GDI-023 ((1R,2S,5S)-N-(cyano(isoquinolin-4-yl) methyl)-3-((S)-3,3-dimethyl-2-((4-methylphenyl)sulfonamido)butanoyl)-6,6-dimethyl-3-azabic yclo[3.1.0]hexane-2-carboxamide)

Compound GDI-023 was obtained in a method similar to that in Example 5.

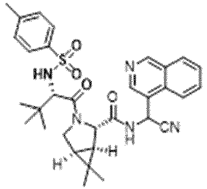

¹H NMR (400 MHz, DMSO-*d*₆) δ 9.62 - 9.51 (m, 1H), 9.42 (d, J = 3.0 Hz, 1H), 8.71 (d, J = 6.4 Hz, 1H), 8.25 (dd, J = 8.9, 4.1 Hz, 1H), 8.03 - 7.72 (m, 4H), 7.66 (dd, J = 12.6, 8.1 Hz, 2H), 7.31 (t, J = 8.8 Hz, 2H), 6.90 - 6.80 (m, 1H), 4.08 (d, J = 9.0 Hz, 1H), 3.84 (dt, J = 13.1, 6.7 Hz, 1H), 3.65 (d, J = 8.4 Hz, 1H), 3.53 - 3.40 (m, 1H), 2.34 (d, J = 3.8 Hz, 3H), 1.58 - 1.48 (m, 1H), 1.35 - 1.18 (m, 1H), 1.06 - 0.80 (m, 12H), 0.54 (d, J = 11.5 Hz, 3H).

### Example 24: Preparation of compound GDI-024 ((1R,2S,5S)-3-((S)-2-acetamido-3,3-dimethylbutyryl)-N-(cyano(1H-indol-3-yl)methyl)-6,6-dimethyl-3-azabicyclo[3.1.0]hexane-2-c arboxamide)

Compound GDI-024 was obtained in a method similar to that in Example 5.

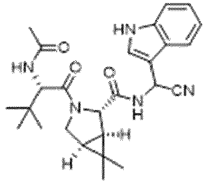

¹H NMR (400 MHz, DMSO-*d*₆) δ 11.27 (s, 1H), 9.17 (dd, J = 13.6, 7.5 Hz, 1H), 7.83 (t, J = 8.0 Hz, 1H), 7.55 (d, J = 7.9 Hz, 1H), 7.49 (dd, J = 6.1, 2.5 Hz, 1H), 7.39 (dd, J = 8.2, 4.3 Hz, 1H), 7.12 (tdd, J = 7.0, 3.2, 1.2 Hz, 1H), 7.06 - 6.95 (m, 1H), 6.25 (dd, J = 7.6, 1.8 Hz, 1H), 4.29 (dd, J = 8.7, 1.7 Hz, 1H), 4.24 (d, J = 2.6 Hz, 1H), 3.86 - 3.76 (m, 2H), 1.80 (d, J = 6.6 Hz, 3H), 1.53 - 1.41 (m, 1H), 1.29 - 1.07 (m, 1H), 0.99 (d, 44 Hz, 3H), 0.93 (s, 9H), 0.79 (d, 8 Hz, 3H).

### Example 25: Preparation of compound GDI-025 ((1R,2S,5S)-N-((5-bromopyridin-3-yl) (cyano)methyl)-3-((S)-3,3-dimethyl-2-((trifluoromethyl)sulfonamido)butanoyl)-6,6-dimethyl-3 -azabicyclo[3.1.0]hexane-2-carboxamide)

Compound GDI-025 was obtained in a method similar to that in Example 5.

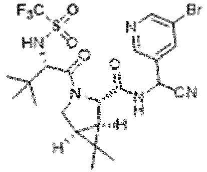

¹H NMR (400 MHz, DMSO-*d*₆) δ 9.81 - 9.50 (m, 2H), 8.89 - 8.60 (m, 2H), 8.10 (dt, J = 12.6, 2.2 Hz, 1H), 6.46 - 6.23 (m, 1H), 4.28 (d, J = 4.3 Hz, 1H), 3.89 (d, J = 8.0 Hz, 2H), 3.63 - 3.47 (m, 1H), 1.64 - 1.54 (m, 1H), 1.45 - 1.31 (m, 1H), 1.04 (d, J = 12.8 Hz, 3H), 1.0 - 0.96 (m, 9H), 0.90 (d, J = 5.6 Hz, 3H).

### Example 26: Preparation of compound GDI-026 ((1R,2S,5S)-N-((5-bromopyridin-3-yl) (cyano)methyl)-3-((S)-2-((4-(dimethylamino)phenyl)sulfonamido)-3,3-dimethylbutanoyl)-6,6-d imethyl-3-azabicyclo[3.1.0]hexane-2-carboxamide)

Compound GDI-026 was obtained in a method similar to that in Example 5.

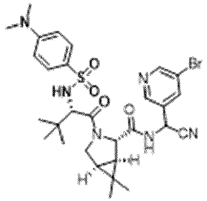

¹H NMR (400 MHz, DMSO-*d*₆) δ 9.66 - 9.52 (m, 1H), 8.76 (dd, J = 8.0, 4.0 Hz, 1H), 8.66 (dd, J = 8.0, 4.0 Hz, 1H), 8.10 (q, J = 2.3 Hz, 1H), 7.68 - 7.47 (m, 2H), 7.27 (dd, J = 11.5, 8.9 Hz, 1H), 6.78 - 6.65 (m, 2H), 6.45 - 6.22 (m, 1H), 4.15 (d, J = 2.1 Hz, 1H), 3.85 - 3.77 (m, 1H), 3.62 - 3.40 (m, 2H), 2.99 (s, 6H), 1.59 - 1.49 (m, 1H), 1.37 - 1.22 (m, 1H), 1.00 (d, J = 15.0 Hz, 3H), 0.82 (d, J = 3.1 Hz, 9H), 0.70 (d, J = 14.5 Hz, 3H).

### Example 27: Preparation of compound GDI-027 ((1R,2S,5S)-N-(cyano(isoquinolin-4-yl) methyl)-3-((S)-2-((4-(dimethylamino)phenyl)sulfonamido)-3,3-dimethylbutyryl)-6,6-dimethyl-3-azabicyclo[3.1.0]hexane-2-carboxamide)

Compound GDI-027 was obtained in a method similar to that in Example 5.

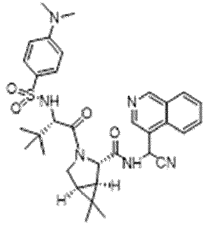

¹H NMR (400 MHz, DMSO-*d*₆) δ 9.62 - 9.50 (m, 1H), 9.42 (d, J = 2.2 Hz, 1H), 8.72 (d, J = 5.9 Hz, 1H), 8.34 - 8.23 (m, 1H), 8.05 - 7.70 (m, 3H), 7.66 - 7.48 (m, 2H), 7.32 - 7.22 (m, 1H), 6.85 (dd, J = 15.3, 7.8 Hz, 1H), 6.76 - 6.54 (m, 2H), 4.11 (d, J = 10.0 Hz, 1H), 3.93 - 3.73 (m, 1H), 3.54 (dd, J = 9.1, 3.4 Hz, 1H), 3.46 (dd, J = 10.1, 6.5 Hz, 1H), 2.97 (s, 6H), 1.60 - 1.48 (m, 1H), 1.28 (d, J = 7.7 Hz, 1H), 1.01 (d, J = 2.7 Hz, 2H), 0.96 - 0.72 (m, 10H), 0.64 (d, J = 10.6 Hz, 3H).

### Example 28: Preparation of compound GDI-028 ((1R,2S,5S)-N-((5-(1H-pyrazol-1-yl) pyridin-3-yl)(cyano)methyl)-3-((S)-2-acetylamino-3,3-dimethylbutanoyl)-6,6-dimethyl-3-azabi cyclo[3.1.0]hexane-2-carboxamide)

Compound GDI-028 was obtained in a method similar to that in Example 5.

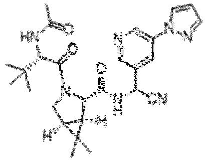

¹H NMR (400 MHz, DMSO-*d*₆) δ 9.60 - 9.50 (m, 1H), 9.17 - 9.14 (m, 1H), 8.73 - 8.65 (m, 1H), 8.62 - 8.59 (m, 1H), 8.34 (q, J = 2.6 Hz, 1H), 7.97 - 7.70 (m, 2H), 6.66 - 6.62 (m, 1H), 6.50 - 6.35 (m, 1H), 4.32 - 4.20 (m, 2H), 3.90 - 3.78 (m, 2H), 1.85 (s, 3H), 1.60 - 1.48 (m, 1H), 1.40 - 1.26 (m, 1H), 1.02 (d, J = 17.3 Hz, 3H), 0.94 - 0.83 (m, 12H).

### Example 29: Preparation of compound GDI-029 ((1R,2S,5S)-N-(cyano(isoquinolin-4-yl) methyl)-3-((S)-3,3-dimethyl-2-((trifluoromethyl)sulfonamido)butanoyl)-6,6-dimethyl-3-azabic yclo[3.1.0]hexane-2-carboxamide)

Compound GDI-029 was obtained in a method similar to that in Example 5.

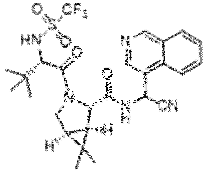

¹H NMR (400 MHz, DMSO-*d*₆) δ 9.76 - 9.52 (m, 2H), 9.42 (s, 1H), 8.79 - 8.69 (m, 1H), 8.35 - 8.20 (m, 1H), 8.10 - 7.65 (m, 3H), 6.87 - 6.75 (m, 1H), 4.23 (d, J = 14.5 Hz, 1H), 4.08 (d, J = 218.1 Hz, 3H), 4.0 - 3.80 (m, 2H), 1.7 - 1.5 (m, 1H), 1.43 - 1.31 (m, 1H), 1.21 - 1.27 (m, 2H), 1.03 - 0.98 (m, 9H), 0.94 - 0.80 (m, 4H).

### Example 30: Preparation of compound GDI-030 ((1R,2S,5S)-3-((S)-2-acetamido-3,3-dimethylbutyryl)-N-(cyano(5-(1-methyl-1H-pyrazol-4-yl)pyridin-3-yl)methyl)-6,6-dimethyl-3-azabicyclo[3.1.0]hexane-2-carboxamide)

Compound GDI-030 was obtained in a method similar to that in Example 5.

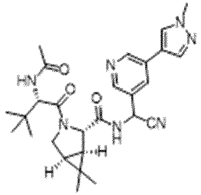

¹H NMR (400 MHz, DMSO-*d*₆) δ 9.54 - 9.38 (m, 1H), 8.86 (d, J = 2.7 Hz, 1H), 8.49 (d, J = 6.5 Hz, 1H), 8.33 (d, J = 20.3 Hz, 1H), 8.06 - 7.95 (m, 2H), 7.86 (t, J = 8.4 Hz, 1H), 6.41 - 6.20 (m, 1H), 4.30 - 4.22 (m, 2H), 4.04 - 3.69 (m, 5H), 1.86 (s, 3H), 1.60 - 1.48 (m, 1H), 1.41 - 1.20 (m, 1H), 1.06 - 0.98 (m, 3H), 0.97 - 0.77 (m, 12H).

### Example 31: Preparation of compound GDI-031 ((1R,2S,5S)-3-((S)-2-acetamido-3,3-dimethylbutyryl)-N-(cyano(5-(3-isopropyl-1,2,4-oxadiazol-5-yl)pyridin-3-yl)methyl)-6,6-dimet hyl-3-azabicyclo[3.1.0]hexane-2-carboxamide)

Compound GDI-031 was obtained in a method similar to that in Example 5.

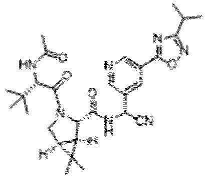

¹H NMR (400 MHz, DMSO-*d*₆) δ 9.64 - 9.52 (m, 1H), 9.26 (s, 1H), 8.93 (d, J = 7.7 Hz, 1H), 8.63 - 8.45 (m, 1H), 7.84 (t, J = 9.0 Hz, 1H), 6.55 - 6.41 (m, 1H), 4.29 - 4.19 (m, 2H), 3.90 - 3.75 (m, 2H), 3.25 - 3.07 (m, 1H), 1.83(s, 3H), 1.62- 1.50 (m, 1H), 1.44 - 1.32 (m, 7H), 1.02 (d, J = 14.0 Hz, 3H), 0.98 - 0.72 (m, 12H).

### Example 32: Preparation of compound GDI-032 ((1R,2S,5S)-N-((5-(1H-pyrazol-4-yl) pyridin-3-yl)(cyano)methyl)-3-((S)-2-acetylamino-3,3-dimethylbutanoyl)-6,6-dimethyl-3-azabi cyclo[3.1.0]hexane-2-carboxamide)

Compound GDI-032 was obtained in a method similar to that in Example 5.

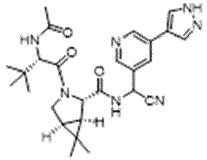

¹H NMR (400 MHz, DMSO-*d*₆) δ 13.13 (s, 1H), 9.52 - 9.38 (m, 1H), 8.91 (s, 1H), 8.59 - 8.30 (m, 2H), 8.15 - 8.00 (s, 2H), 7.90 - 7.80 (m, 1H), 6.38 - 8.20 (m, 1H), 4.41 - 4.18 (m, 2H), 3.90 - 3.78 (m, 2H), 1.85 (s, 3H), 1.69 - 1.51 (m, 1H), 1.43 - 1.22 (m, 1H), 1.11 - 0.75 (m, 15H).

### Example 33: Preparation of compound GDI-033 ((1R,2S,5S)-3-((S)-2-acetamido-3,3-dimethylbutyryl)-N-(cyano(8-fluoroisoquinolin-4-yl)methyl)-6,6-dimethyl-3-azabicyclo[3.1.0]h exane-2-carboxamide)

Compound GDI-033 was obtained in a method similar to that in Example 5.

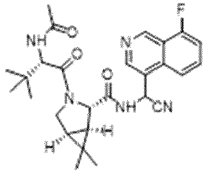

¹H NMR (400 MHz, DMSO-*d*₆) δ 9.72 - 9.33 (m, 2H), 8.83 (d, J = 8.1 Hz, 1H), 8.06 - 7.73 (m, 3H), 7.61 (t, J = 9.1 Hz, 1H), 6.89 (dd, J = 11.0, 8.1 Hz, 1H), 4.27 (t, J = 8.2 Hz, 1H), 4.19 (d, J = 13.4 Hz, 1H), 3.93 - 3.79 (m, 2H), 1.82 (d, J = 9.8 Hz, 3H), 1.60 - 1.48 (m, 1H), 1.34 - 1.07 (m, 1H), 1.06 - 0.76 (m, 15H).

### Example 34: Preparation of compound GDI-034 (5-((1R,2S,5S)-3-((S)-2-acetamido-3,3-dimethylbutyryl)-6,6-dimethyl-3-azabicyclo[3.1.0]hexane-2-carboxamido)(cyano)methyl)meth yl nicotinate)

Compound GDI-034 was obtained in a method similar to that in Example 5.

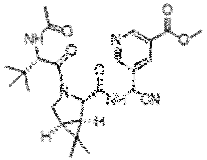

¹H NMR (400 MHz, DMSO-*d*₆) δ 9.59 - 9.48 (m, 1H), 9.09 (s, 1H), 8.89 (dd, J = 6.0, 2.8 Hz, 1H), 8.38 (s, 1H), 7.85 (t, J = 8.0 Hz, 1H), 6.52 - 6.36 (m, 1H), 4.29 - 4.19 (m, 2H), 3.92 (s, 3H), 3.87 - 3.76 (m, 2H), 2.07 (s, 1H), 1.84 (s, 3H), 1.65 - 1.42 (m, 1H), 1.37 - 1.22 (m, 1H), 1.02 (d, J = 13.6 Hz, 3H), 0.88 (m, 12H).

### Example 35: Preparation of compound GDI-035 ((1R,2S,5S)-N-((5-(1H-pyrazol-5-yl) pyridin-3-yl)(cyano)methyl)-3-((S)-2-acetylamino-3,3-dimethylbutanoyl)-6,6-dimethyl-3-azabi cyclo[3.1.0]hexane-2-carboxamide)

Compound GDI-035 was obtained in a method similar to that in Example 5.

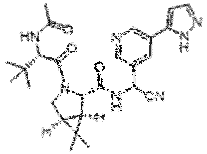

¹H NMR (400 MHz, DMSO-*d*₆) δ 13.15 (s, 1H), 9.53 - 9.45 (m, 1H), 9.04 (s, 1H), 8.66 - 8.52 (m, 1H), 8.28 (s, 1H), 7.85 (d, J = 8.0 Hz, 2H), 6.93 - 6.85 (m, 1H), 6.43 - 6.30 (m, 1H), 4.32 - 4.23 (m, 2H), 3.88 - 3.80 (m, 2H), 1.85 (s, 3H), 1.60 - 1.48 (m, 1H), 1.38 - 1.22 (m, 1H), 1.12 - 0.81 (m, 15H).

### Example 36 and Example 37: Preparation of compound GDI-036 and compound GDI-037

The compound 6 was chirally split to obtain compounds GDI-036 and GDI-037.

YMC Cellulose-SC (10 µm 50*250 mm) was used as a chiral column, and hexane and ethanol in a ratio of 7:3 were used as a mobile phase. A separation volume was 300 mg, and retention periods were 2 minutes and 7.5 minutes separately.

¹H NMR (400 MHz, DMSO-d₆) δ = 9.44 (d, *J* = 7.9 Hz, 1H), 9.42 - 9.39 (m, 1H), 8.73 - 8.69 (m, 1H), 8.27 - 8.22 (m, 1H), 8.02 - 7.97 (m, 1H), 7.94 - 7.89 (m, 1H), 7.88 - 7.83 (m, 1H), 7.81 - 7.76 (m, 1H), 6.87 - 6.83 (m, 1H), 4.30 - 4.25 (m, 1H), 4.23 - 4.19 (m, 1H), 3.90 -F 3.83 (m, 1H), 3.82 - 3.76 (m, 1H), 1.87 - 1.79 (m, 3H), 1.54 - 1.46 (m, 1H), 1.09 - 1.05 (m, 1H), 0.92 - 0.87 (m, 12H), 0.83 - 0.77 (m, 3H).

¹H NMR (400 MHz, DMSO-d₆) δ = 9.60 - 9.50 (m, 1H), 9.45 - 9.36 (m, 1H), 8.77 - 8.68 (m, 1H), 8.26 - 8.19 (m, 1H), 8.00 - 7.93 (m, 1H), 7.85 - 7.73 (m, 3H), 6.94 - 6.84 (m, 1H), 4.33 - 4.25 (m, 1H), 4.19 - 4.13 (m, 1H), 3.94 - 3.85 (m, 1H), 3.84 - 3.77 (m, 1H), 1.83 - 1.77 (m, 3H), 1.61 - 1.53 (m, 1H), 1.34 - 1.27 (m, 1H), 1.02 (s, 3H), 0.95 (s, 9H), 0.82 (s, 3H).

### Test Example 1: Measurement of compounds' activities

### SARS-CoV-2/Hela-ACE Test

The compound was transferred to a 384-well plate (Greiner, Part. No. 781090-2B), and then Hela-ACE cells were added (cell density: 5000 cells/20 microliter of medium MEM with 2% FBS). The plate with cells was transferred to BSL3 laboratory. SARS-CoV-2 (USA-WA1/2020 Vero E6 cell proliferation) was diluted to MOI 0.75-1 until 30%-60% cells were infected. After the assay plate was incubated at 37°C in 5% CO₂ for 48 hours, formaldehyde was added until reaching a final concentration of 4% to fix the cells. A human polyclonal serum antibody was used as a primary antibody, and goat anti-human H+L conjugated Alexa 488 (Thermo Fisher Scientific A11013) was used as a secondary antibody. DAPI (Thermo Fisher Scientific D1306) was used for DNA staining.

### Toxicity Testing in Uninfected Cells

The compound was transferred to a 1536-well plate (Corning No. 9006BC), and then Hela-ACE cells were added (cell density: 600 cells/5 microliter of medium MEM with 2% FBS). After the assay plate was incubated at 37°C in 5% CO₂ for 48 hours, cell viability was tested. 2 µL of 50% Cell-Titer Glo (Promega No G7573) was diluted in water and added to a cell assay plate, and then a value on EnVision Plate Reader (Perkin Elmer) was read.

### Enzyme Activity Test

An inhibition test was conducted using 200 nM recombinant SARS-CoV-2 main protease and 15 µM fluorescent substrate (Dabcyl-TSAVL QSGFRK-Glu (EDANS); Genscript). An assay buffer consisted of 50 mM Tris-HCl and 1 mM EDTA at pH 7.3. SARS-CoV-2 3CLpro was dissolved in 25 µL of the assay buffer and mixed with compounds at different concentrations. The mixture was incubated at 37°C for 30 minutes. A substrate dissolved in 25 µL of the assay buffer was then added to initiate a reaction. Fluorescent signals at 350 nm (excitation)/490 nm (emission) were measured immediately each minute in 10 minutes at 37°C using a SpectraMax^{®} M5 multimode microplate reader. RFU at 6^{th} minute of the reaction with the compounds at different concentrations in contrast with a reaction with the lowest concentration was used to generate an IC₅₀ curve. IC₅₀ values corresponding to SARS-CoV-2 3CLpro were measured for each compound at 12 concentrations. Test data were analyzed using GraphPad Prism software.

Results of the activities of the exemplary compounds are shown in Table 1 below.

**Table 1: Results of the activities of the Exemplary Compound**

| **Example** | **No.** | **Structure** | **SAR-CoV-2 EC₅₀ (µM)** | **Uninfected Hela-ACE CC₅₀ (µM)** | **IC₅₀ (µM)** |
|---|---|---|---|---|---|
| 1 | GDI-001 | 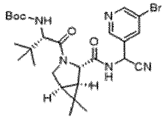 | 0.462 | >39.8 | 0.080 |
| 2 | GDI-002 | 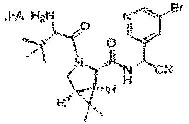 | 7.859 | >39.8 | 0.32 |
| 3 | GDI-003 | 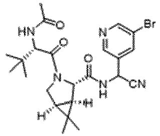 | 1.253 | >39.8 | 0.05 |
| 4 | GDI-004 | 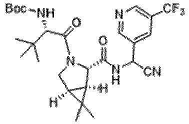 | 0.547 | >39.8 | 0.027 |
| 5 | GDI-005 | 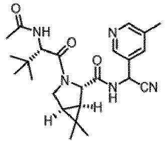 | 0.117 | >39.8 | 0.080 |
| 6 | GDI-006 | 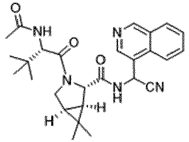 | 0.028 | >39.8 | 0.028 |
| 7 | GDI-007 | 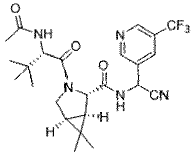 | 0.512 | >39.8 | 0.17 |
| 8 | GDI-008 | 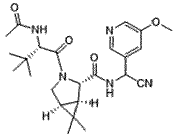 | 0.417 | >39.8 | 0.037 |
| 9 | GDI-009 | 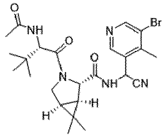 | - | - | 0.053 |
| 10 | GDI-010 | 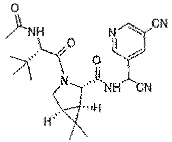 | 5.719 | >39.8 | 0.33 |
| 11 | GDI-011 | 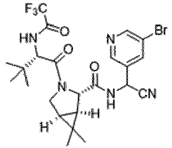 | 0.613 | >39.8 | 0.078 |
| 12 | GDI-012 | 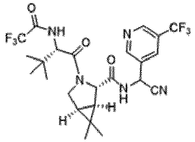 | - | - | 0.024 |
| 13 | GDI-013 | 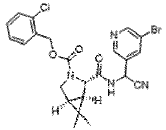 | - | - | 0.34 |
| 14 | GDI-014 | 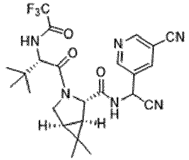 | - | - | 0.046 |
| 15 | GDI-015 | 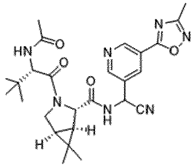 | - | - | 0.12 |
| 16 | GDI-016 | 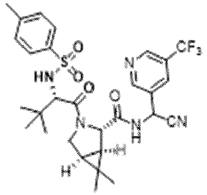 | 0.032 | >39.8 | 0.031 |
| 17 | GDI-017 | 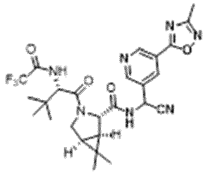 | 0.66 | >39.8 | 0.046 |
| 18 | GDI-018 | 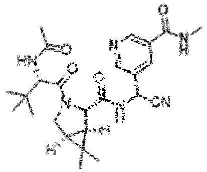 | - | - | 0.11 |
| 19 | GDI-019 | 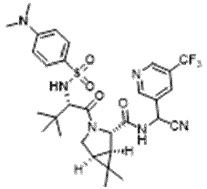 | - | - | 0.022 |
| 20 | GDI-020 | 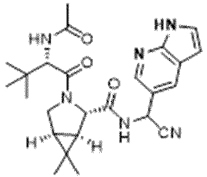 | - | - | 0.019 |
| 21 | GDI-021 | 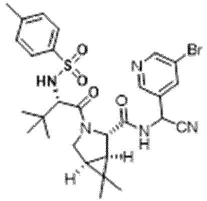 | - | - | 0.019 |
| 22 | GDI-022 | 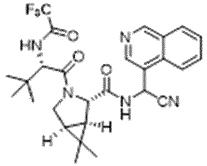 | - | - | 0.017 |
| 23 | GDI-023 | 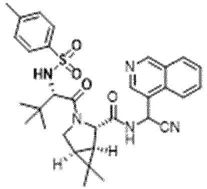 | - | - | 0.015 |
| 24 | GDI-024 | 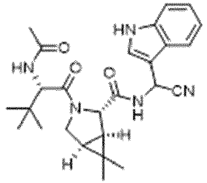 | - | - | 66.29 |
| 25 | GDI-025 | 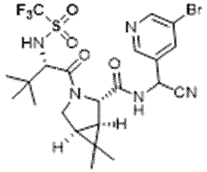 | - | - | 0.019 |
| 26 | GDI-026 | 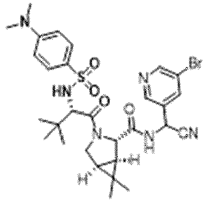 | - | - | 0.017 |
| 27 | GDI-027 | 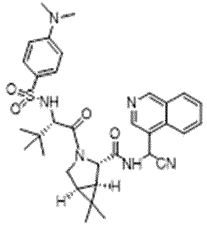 | - | - | 0.0076 |
| 28 | GDI-028 | 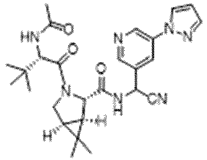 | - | - | 0.0024 |
| 29 | GDI-029 | 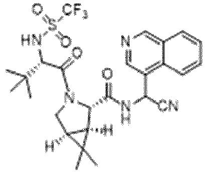 | - | - | 0.023 |
| 30 | GDI-030 | 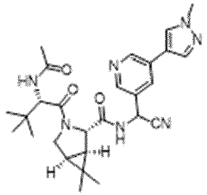 | - | - | 0.018 |
| 31 | GDI-031 | 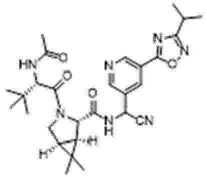 | - | - | 0.10 |
| 32 | GDI-032 | 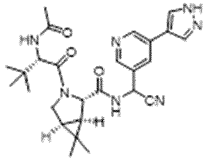 | - | - | 0.028 |
| 33 | GDI-033 | 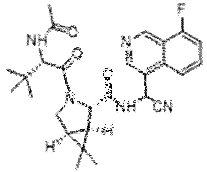 | - | - | 0.056 |
| 34 | GDI-034 | 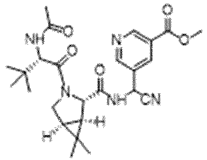 | - | - | 0.062 |
| 35 | GDI-035 | 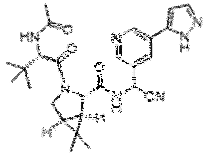 | - | - | 0.017 |
| 36 | GDI-036 | 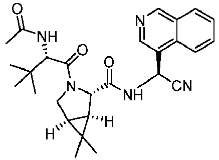 | 0.02 | >39.8 | 0.02 |
| 37 | GDI-037 | 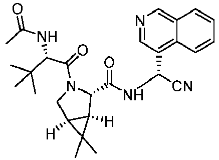 | 0.03 | >39.8 | 0.04 |

### Test Example 2: Measurement of Oral Pharmacokinetics in Rats

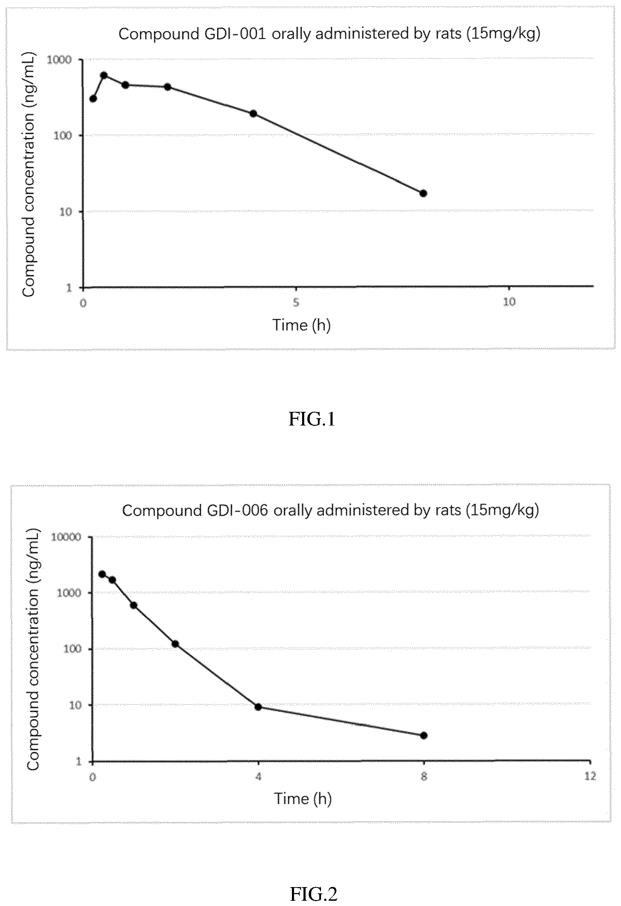

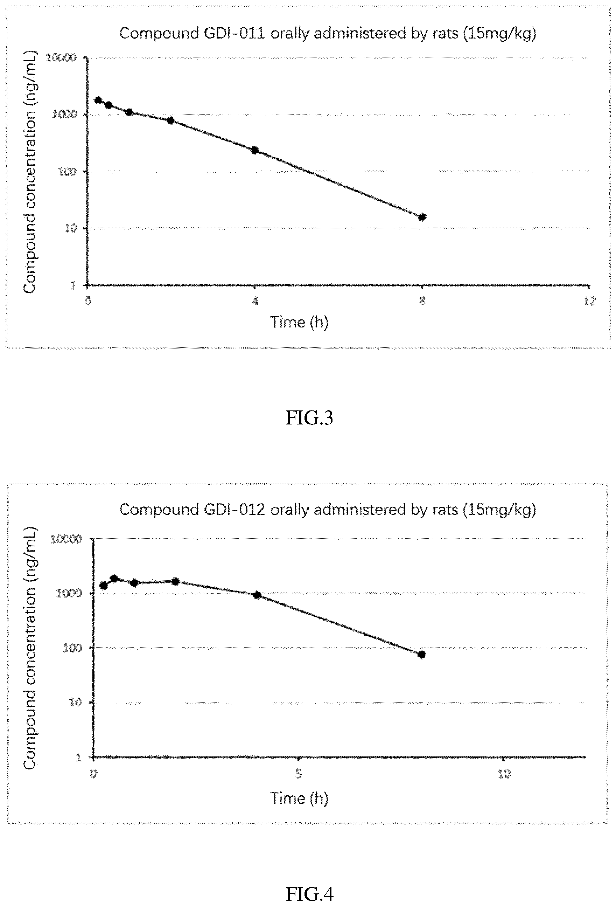

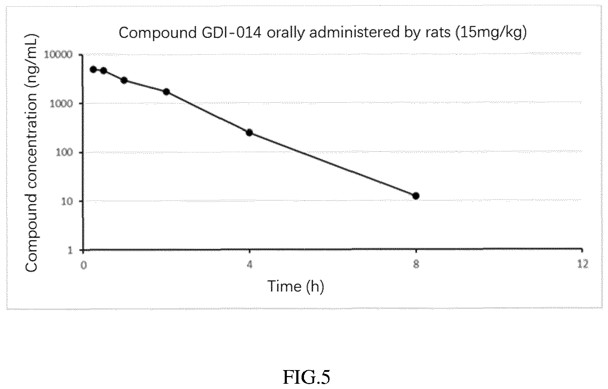

Male SD rats were orally administered with 15 mg/kg of the compound in the examples of the present disclosure, and blood samples were collected at 0.083, 0.25, 0.5, 1, 2, 4, and 8 hours respectively. After the samples were treated with anticoagulant EDTA-K2 and acetonitrile and centrifuged, a drug concentration analysis was conducted using LC/MS/MS. Test results are shown in Table 1 and FIG. 1 to FIG. 5. FIG. 1 shows a PK diagram of compound GDI-001 orally administered to rats; FIG. 2 shows a PK diagram of compound GDI-006 orally administered to rats; FIG. 3 shows a PK diagram of compound GDI-011 orally administered to rats; FIG. 4 shows a PK diagram of compound GDI-012 orally administered to rats; and FIG. 5 shows a PK diagram of compound GDI-014 orally administered to rats.

**Table 1**

| Example | No. | Cₘₐₓ (ng/mL) | T_{1/2} (h) | AUC₀₋ₗₐₛₜ (ng.h/mL) | AUC_{0-inf} (ng.h/mL) |
|---|---|---|---|---|---|
| 1 | GDI-001 | 630 | 2.34 | 1813 | 1829 |
| 6 | GDI-006 | 2174 | 0.875 | 1692 | 1695 |
| 11 | GDI-011 | 1867 | 1.05 | 3450 | 3474 |
| 12 | GDI-012 | 2121 | 1.92 | 7075 | 7139 |
| 14 | GDI-014 | 4997 | 0.832 | 7801 | 7817 |

## Claims

1. A compound of formula (I) or an isotope-labeled compound thereof, or an optical isomer, a geometric isomer, a tautomer, or an isomer mixture thereof, or a pharmaceutically acceptable salt thereof, or a prodrug thereof, or a metabolite thereof; 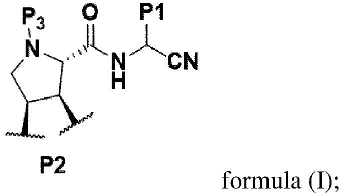 wherein:
P1 is a five- to ten-membered heteroaryl group that is unsubstituted or optionally substituted by a group R1, wherein the heteroaryl group contains at least one heteroatom selected from N, O, or S;
P2 together with two adjacent carbon atoms jointly form a three-membered, four-membered, five-membered, or six-membered cycloalkyl group or heterocycloalkyl group that is unsubstituted or optionally substituted by a C₁-C₃ alkyl group or halogen, wherein the heterocycloalkyl group contains at least one heteroatom selected from N, O, or S; and
P3 is selected from 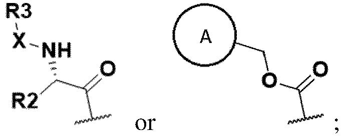
further, wherein:
R1 is selected from hydrogen, deuterium, halogen, a cyano group, an oxo group, a nitro group, a C₁-C₅ alkyl group, a C₁-C₃ haloalkyl group, a C₁-C₄ alkoxy group, a C₅-C₁₀ aryl group, or a five-to ten-membered heteroaryl group;
R2 is selected from a C₁-C₅ alkyl group, a C₁-C₃ haloalkyl group, a C₃-C₆ cycloalkyl group, a C₁-C₄ alkoxy group, or a C₅-C₁₀ aryl group;
R3 is selected from a C₁-C₅ alkyl group, a C₁-C₃ haloalkyl group, a C₃-C₆ cycloalkyl group, a C₁-C₄ alkoxy group, a C₅-C₁₀ aryl group, or a five- to ten-membered heteroaryl group;
ring A is selected from a C₅-C₁₀ aryl group or a five- to ten-membered heteroaryl group that is unsubstituted or optionally substituted by halogen, wherein the heteroaryl group contains at least one heteroatom selected from N, O, or S; and
X is selected from

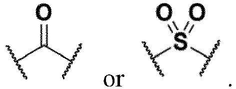

2. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of claim 1, wherein the compound of formula (I) is of formula (II):

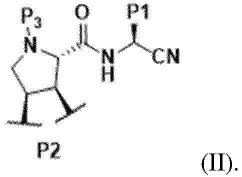

3. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of claim 1, wherein the compound of formula (I) is of formula (III):

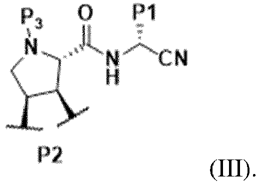

4. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 3, wherein:
P1 is a five- to six-membered monocyclic or eight- to ten-membered bicyclic heteroaryl group that is unsubstituted or optionally substituted by a group R1, wherein the heteroaryl group contains one to three heteroatoms selected from N, O, or S;
P2 together with two adjacent carbon atoms jointly form a three-membered, four-membered, five-membered, or six-membered cycloalkyl group or heterocycloalkyl group that is unsubstituted or optionally substituted by a methyl group, an ethyl group, fluorine, or chlorine, wherein the heterocycloalkyl group contains one or two heteroatoms selected from N, O, or S; and
P3 is selected from 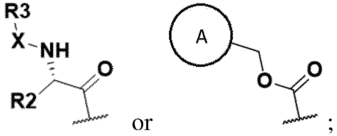
further, wherein:
R1 is selected from hydrogen, deuterium, halogen, a cyano group, an oxo group, a nitro group, a C₁-C₅ alkyl group, a C₁-C₃ haloalkyl group, a C₁-C₄ alkoxy group, a C₅-C₁₀ aryl group, or a five-to ten-membered heteroaryl group;
R2 is selected from a C₁-C₅ alkyl group, a C₁-C₃ haloalkyl group, a C₃-C₆ cycloalkyl group, a C₁-C₄ alkoxy group, or a C₅-C₁₀ aryl group;
R3 is selected from a C₁-C₅ alkyl group, a C₁-C₃ haloalkyl group, a C₃-C₆ cycloalkyl group, a C₁-C₄ alkoxy group, a C₅-C₁₀ aryl group, or a five- to ten-membered heteroaryl group;
ring A is selected from a phenyl group, a pyridyl group, a pyrimidinyl group, a pyrrolyl group, a furyl group, or a thienyl group that is unsubstituted or optionally substituted by halogen; and
X is selected from

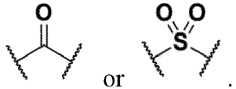

5. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 3, wherein:
P1 is selected from 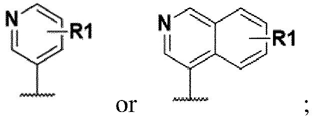
P2 is selected from 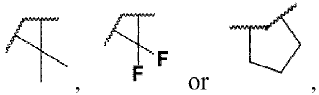 and P2 together with two adjacent carbon atoms jointly form a cyclopropyl group substituted by two methyl groups, a cyclopropyl group substituted by two fluorine atoms, or a cyclopentyl group; and
P3 is selected from 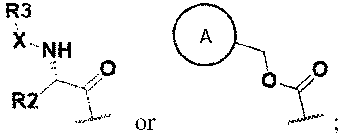
further, wherein:
R1 is selected from hydrogen, deuterium, halogen, a cyano group, a nitro group, a C₁-C₅ alkyl group, a C₁-C₃ haloalkyl group, a C₁-C₄ alkoxy group, a C₅-C₁₀ aryl group, or a five- to ten-membered heteroaryl group;
R2 is selected from a C₁-C₅ alkyl group, a C₁-C₃ haloalkyl group, a C₃-C₆ cycloalkyl group, a C₁-C₄ alkoxy group, or a C₅-C₁₀ aryl group;
R3 is selected from a C₁-C₅ alkyl group, a C₁-C₃ haloalkyl group, a C₃-C₆ cycloalkyl group, a C₁-C₄ alkoxy group, a C₅-C₁₀ aryl group, or a five- to ten-membered heteroaryl group;
ring A is selected from a phenyl group that is unsubstituted or optionally substituted by fluorine, chlorine, or bromine; and
X is selected from

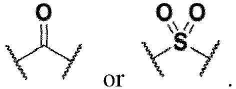

6. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 3, wherein:
R1 is selected from hydrogen, deuterium, halogen, a cyano group, a nitro group, a methyl group, an ethyl group, a propyl group, an isopropyl group, a halomethyl group, a haloethyl group, a halopropyl group, a methoxy group, an ethoxy group, a propoxy group, a tert-butoxy group, a phenyl group, a halophenyl group, or a pyridyl group;
R2 is selected from a methyl group, an ethyl group, a propyl group, an isopropyl group, a butyl group, a tert-butyl group, a halomethyl group, a haloethyl group, a halopropyl group, a cyclopropyl group, a cyclobutyl group, a cyclopentyl group, a cyclohexyl group, a methoxy group, an ethoxy group, a propoxy group, a tert-butoxy group, a phenyl group, or a halophenyl group; and
R3 is selected from a methyl group, an ethyl group, a propyl group, an isopropyl group, a butyl group, a tert-butyl group, a halomethyl group, a haloethyl group, a halopropyl group, a cyclopropyl group, a cyclobutyl group, a cyclopentyl group, a cyclohexyl group, a methoxy group, an ethoxy group, a propoxy group, a tert-butoxy group, a phenyl group, a halophenyl group, or a pyridyl group.

7. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 3, wherein:
R1 is selected from hydrogen, bromine, a cyano group, a methyl group, a fluoromethyl group, a methoxy group, a phenyl group, a benzyl group, a pyridyl group, or a pyrazolyl group;
R2 is selected from an isopropyl group, a tert-butyl group, a phenyl group, or a chlorophenyl group; and
R3 is selected from a methyl group, a trifluoromethyl group, a cyclopropyl group, a tert-butoxy group, a tolyl group, a chlorophenyl group, an aminophenyl group, or an amidophenyl group.

8. The compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 3, wherein the compound of formula (I) is of the following formulas: 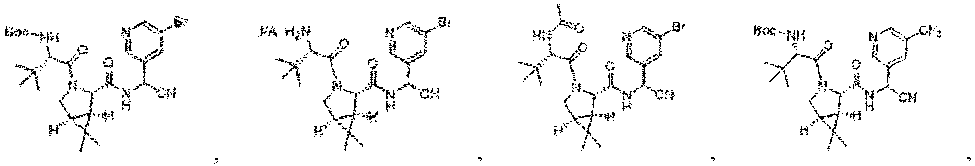 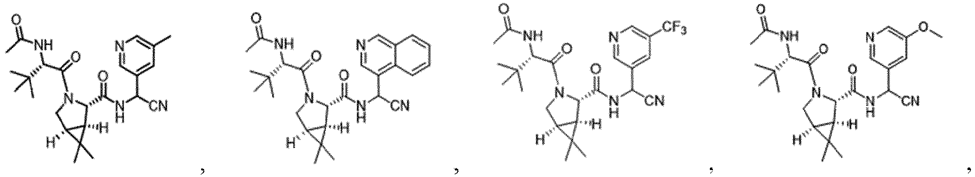 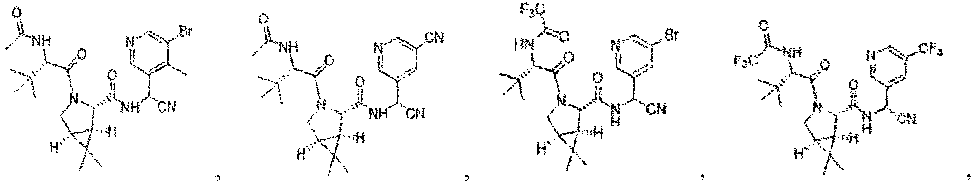 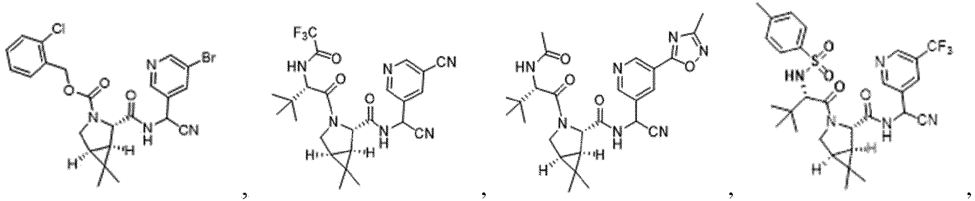 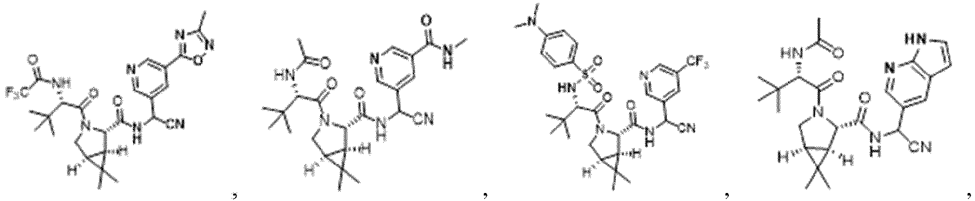 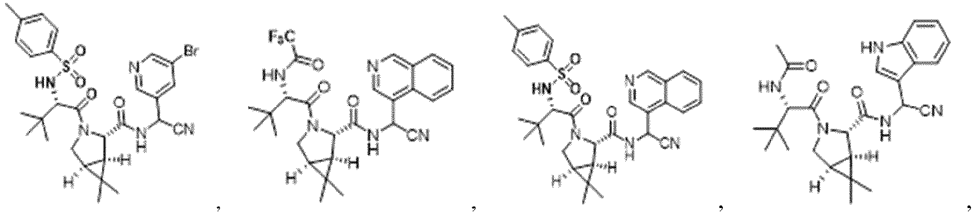 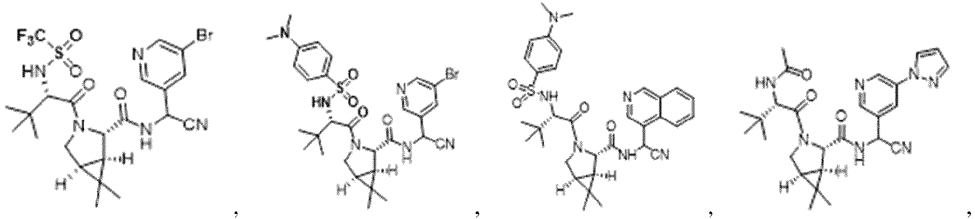 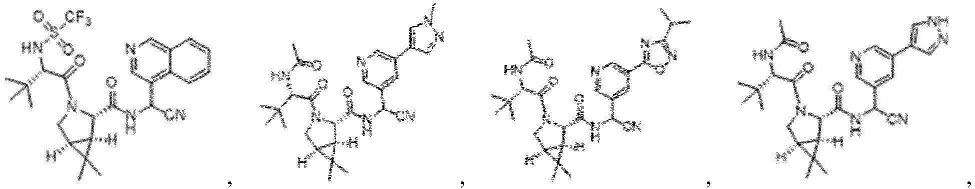 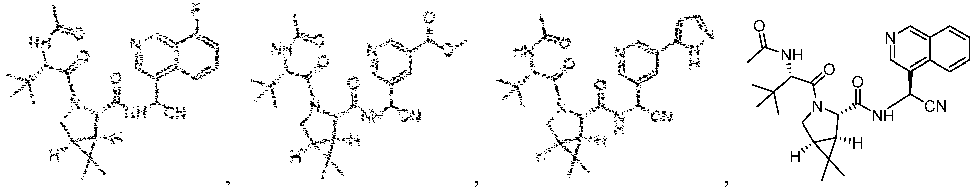 or

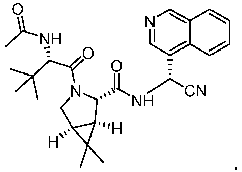

9. A pharmaceutical composition, comprising the compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 8, and a pharmaceutically acceptable carrier.

10. The pharmaceutical composition of claim 9, wherein a dosage form of the pharmaceutical composition comprises a tablet, granules, powder, syrup, an inhalation, and an injection.

11. Use of the compound of formula (I) or the isotope-labeled compound thereof, or the optical isomer, the geometric isomer, the tautomer, the isomer mixture thereof, or the pharmaceutically acceptable salt thereof, or the prodrug thereof, or the metabolite thereof of any one of claims 1 to 8 in the preparation of a medicament for treating or preventing a coronavirus infection or a disease or symptom caused by a coronavirus in a subject in need.

12. The use of claim 11, wherein the coronavirus is selected from severe acute respiratory syndrome coronavirus (SARS-CoV), severe acute respiratory syndrome coronavirus 2 (SARS-CoV -2), Middle East respiratory syndrome coronavirus (MERS-CoV), human coronavirus OC43 (HCoV-OC43), murine hepatitis coronavirus (MHV), and a coronavirus that shares more than 85% homology with any one of the foregoing coronaviruses and that has viral activity.

13. The use of claim 11 or 12, wherein the disease or symptom caused by a coronavirus is selected from one or more of: a respiratory infection, severe acute respiratory syndrome (SARS), pneumonia (including severe pneumonia), gastroenteritis (including acute gastroenteritis), cough, fever, shivering, vomiting, headache, chill, shortness of breath, and cytokine storm.
